(19) Europäisches Patentamt
European Patent Office
Office européen des brevets

(11) EP 0 785 197 A2

(12) **EUROPÄISCHE PATENTANMELDUNG**

(43) Veröffentlichungstag:
**23.07.1997 Patentblatt 1997/30**

(51) Int. Cl.[6]: **C07D 413/04**, A61K 31/49, A61K 31/47

(21) Anmeldenummer: **97100026.0**

(22) Anmeldetag: **03.01.1997**

(84) Benannte Vertragsstaaten:
**AT BE CH DE DK ES FI FR GB GR IE IT LI LU MC NL PT SE**
Benannte Erstreckungsstaaten:
**LT LV RO SI**

(30) Priorität: **16.01.1996 DE 19601265**

(71) Anmelder: **BAYER AG**
**51368 Leverkusen (DE)**

(72) Erfinder:
- **Häbich, Dieter, Dr.**
  **42115 Wuppertal (DE)**
- **Stolle, Andreas, Dr.**
  **42115 Wuppertal (DE)**
- **Riedl, Bernd, Dr.**
  **42329 Wuppertal (DE)**
- **Ruppelt, Martin, Dr.**
  **42115 Wuppertal (DE)**
- **Bartel, Stephan, Dr.**
  **51465 Bergisch Gladbach (DE)**
- **Guarnieri, Walter, Dr.**
  **53909 Zülpich (DE)**
- **Endermann, Rainer, Dr.**
  **42113 Wuppertal (DE)**
- **Kroll, Hein-Peter, Dr.**
  **42115 Wuppertal (DE)**

(54) **2-Oxo- und 2-Thio-1,2-dihydrochinolinyl-oxazolidinone**

(57) Die vorliegende Erfindung betrifft 2-Oxo- und 2-Thio-1,2-dihydrochinolinyl-oxazolidinone, Verfahren zu ihrer Herstellung und ihre Verwendung als Arzneimittel, insbesondere als antibakterielle Arzneimittel.

EP 0 785 197 A2

Printed by Rank Xerox (UK) Business Services
2.14.11/3.4

## Beschreibung

Die vorliegende Erfindung betrifft 2-Oxo- und 2-Thio-1,2-dihydrochinolinyl-oxazolidinone, Verfahren zu ihrer Herstellung und ihre Verwendung als Arzneimittel, insbesondere als antibakterielle Arzneimittel.

N-Aryloxazolidinone mit antibakterieller Wirkung sind beispielsweise aus der Publikation EP 311 090 bekannt. Außerdem sind 3-(Stickstoff-substituierte)phenyl-5-beta-amidomethyloxazolidin-2-one aus der EP 609 905 A1 bekannt.

Ferner sind in der WO 93 08 179 A Oxazolidinonderivate mit einer Monoaminoxidase inhibitorischen Wirkung und in der EP 645 376 mit Wirkung als Adhäsionsrezeptor-Antagonisten publiziert.

Die vorliegende Erfindung betrifft 2-Oxo- und 2-Thio-1,2-dihydrochinolinyl-oxazolidinone der allgemeinen Formel (I)

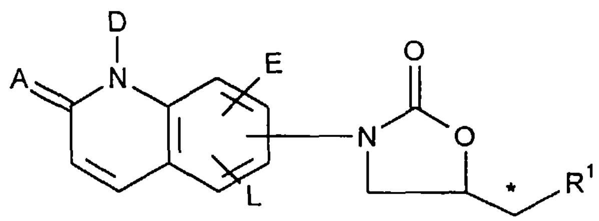

(I) ,

in welcher

A für ein Sauerstoff- oder ein Schwefelatom steht,

D für Wasserstoff oder für Cycloalkyl mit 3 bis 6 Kohlenstoffatomen steht, oder
für geradkettiges oder verzweigtes Alkoxycarbonyl mit bis zu 4 Kohlenstoffatomen steht, oder
für geradkettiges oder verzweigtes Alkyl oder Alkenyl mit jeweils bis zu 9 Kohlenstoffatomen steht, das gegebenenfalls durch Cyano, Trifluormethyl, Halogen, Hydroxy, Pyridyl, Phenyl, Carboxyl, Carboxamido, geradkettiges oder verzweigtes Alkoxycarbonyl mit bis zu 5 Kohlenstoffatomen, Naphthyl, Cycloalkyl mit 3 bis 6 Kohlenstoffatomen, und/oder durch eine Gruppe der Formel $-(CO)_a-NR^2R^3$,

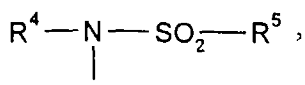

$R^6R^7$-N-$SO_2$- oder $R^8$-$S(O)_b$ substituiert ist,
worin

a eine Zahl 0 oder 1 bedeutet,

$R^2$, $R^3$, $R^4$, $R^6$ und $R^7$ gleich oder verschieden sind und Wasserstoff, geradkettiges oder verzweigtes Alkyl mit bis zu 5 Kohlenstoffatomen oder Phenyl bedeuten, oder

$R^2$ und $R^3$ gemeinsam mit dem Stickstoffatom einen Morpholinyl-, Thiomorpholinyl-, Pyrrolidinyl-, Piperazinyl- oder Piperidylring bilden, die gegebenenfalls, auch über die freie N-Funktion, durch geradkettiges oder verzweigtes Alkyl oder Acyl mit jeweils bis zu 4 Kohlenstoffatomen substituiert sind,

b eine Zahl 0, 1 oder 2 bedeutet,

$R^5$ und $R^8$ gleich oder verschieden sind und geradkettiges oder verzweigtes Alkyl mit bis zu 6 Kohlenstoffatomen, Benzyl, Phenyl oder Tolyl bedeuten, oder

D — für geradkettiges oder verzweigtes Acyl mit bis zu 5 Kohlenstoffatomen steht, das gegebenenfalls durch Trifluormethyl, Trichlormethyl oder eine Gruppe der Formel $-OR^9$ substituiert ist,
worin

$R^9$ — Wasserstoff oder geradkettiges oder verzweigtes Alkyl mit bis zu 5 Kohlenstoffatomen bedeutet, das gegebenenfalls durch Phenyl oder Naphthyl substituiert ist,
oder

D — für eine Gruppe der Formel $-(CT)_d-NR^{10}R^{11}$, $-(CO)_e-NR^{12}-CO-NR^{13}R^{14}$, $-NR^{15}-SO_2R^{16}$, $R^{17}R^{18}-N-SO_2-$, $R^{19}-S(O)_f$ oder $-CO-R^{20}$ steht,
worin

T — ein Sauerstoff- oder Schwefelatom bedeutet,

d und e — gleich oder verschieden sind und die oben angegebene Bedeutung von a haben und mit dieser gleich oder verschieden sind,

$R^{10}$, $R^{11}$, $R^{12}$, $R^{13}$, $R^{14}$, $R^{15}$, $R^{17}$ und $R^{18}$ — die jeweils oben angegebene Bedeutung von $R^2$, $R^3$ und $R^4$ haben und mit dieser gleich oder verschieden sind,

f — die oben angegebene Bedeutung von b hat und mit dieser gleich oder verschieden ist,

$R^{16}$ und $R^{19}$ — gleich oder verschieden sind und die oben angegebene Bedeutung von $R^5$ und $R^8$ haben,

$R^{20}$ — Aryl mit 6 bis 10 Kohlenstoffatomen bedeutet, das gegebenenfalls durch Halogen substituiert ist,

E und L — gleich oder verschieden sind und für Wasserstoff, Carboxy, Halogen, Cyano, Formyl, Trifluormethyl, Nitro, für geradkettiges oder verzweigtes Alkyl mit bis zu 4 Kohlenstoffatomen stehen,

$R^1$ — für Azido, Hydroxy oder für eine Gruppe der Formel $-OR^{21}$, $O-SO_2R^{22}$ oder $-NR^{23}R^{24}$ steht,
worin

$R^{21}$ — geradkettiges oder verzweigtes Acyl mit bis zu 6 Kohlenstoffatomen oder eine Hydroxyschutzgruppe bedeutet,

$R^{22}$ — geradkettiges oder verzweigtes Alkyl mit bis zu 5 Kohlenstoffatomen, Phenyl oder Tolyl bedeutet,

$R^{23}$ und $R^{24}$ — gleich oder verschieden sind und
Cycloalkyl mit 3 bis 6 Kohlenstoffatomen, Wasserstoff, Phenyl oder geradkettiges oder verzweigtes Alkoxy mit bis zu 6 Kohlenstoffatomen, tert.Butoxycarbonyl, Fluorenyloxycarbonyl oder Benzyloxycarbonyl bedeuten, oder
geradkettiges oder verzweigtes Alkyl mit bis zu 6 Kohlenstoffatomen bedeuten, das gegebenenfalls durch Cyano oder Alkoxycarbonyl mit bis zu 4 Kohlenstoffatomen substituiert ist,
oder

$R^{23}$ und/oder $R^{24}$ — eine Gruppe der Formel $-CT'-R^{25}$, $P(O)(OR^{26})(OR^{27})$ oder $-SO_2-R^{28}$ bedeutet,
worin

| | |
|---|---|
| T | die oben angegebene Bedeutung von T hat und mit dieser gleich oder verschieden ist, |
| $R^{25}$ | Cycloalkyl mit 3 bis 6 Kohlenstoffatomen, Trifluormethyl oder geradkettiges oder verzweigtes Alkoxy mit bis zu 6 Kohlenstoffatomen, Phenyl, Benzyloxy oder Wasserstoff bedeutet, oder<br>geradkettiges oder verzweigtes Alkyl mit bis zu 6 Kohlenstoffatomen bedeutet, das gegebenenfalls durch geradkettiges oder verzweigtes Alkoxycarbonyl mit bis zu 5 Kohlenstoffatomen, Cyano, Fluor, Chlor, Brom oder Trifluormethyl substituiert ist, oder<br>geradkettiges oder verzweigtes Thioalkyl oder Acyl mit jeweils bis zu 5 Kohlenstoffatomen bedeutet, oder eine Gruppe der Formel $-NR^{29}R^{30}$ bedeutet, worin |
| $R^{29}$ und $R^{30}$ | gleich oder verschieden sind und Wasserstoff, Phenyl oder geradkettiges oder verzweigtes Alkyl mit bis zu 4 Kohlenstoffatomen bedeuten, oder |
| $R^{26}$ und $R^{27}$ | gleich oder verschieden sind und Wasserstoff oder geradkettiges oder verzweigtes Alkyl mit bis zu 4 Kohlenstoffatomen bedeuten, |
| $R^{28}$ | geradkettiges oder verzweigtes Alkyl mit bis zu 4 Kohlenstoffatomen oder Phenyl bedeutet, |

und deren Salze und Isomere.

Physiologisch unbedenkliche Salze der erfindungsgemäßen Oxazolidinone können Salze mit Mineralsäuren, Carbonsäuren oder Sulfonsäuren sein. Besonders bevorzugt sind z.B. Salze mit Chlorwasserstoffsäure, Bromwasserstoffsäure, Schwefelsäure, Phosphorsäure, Methansulfonsäure, Ethansulfonsäure, Toluolsulfonsäure, Benzolsulfonsäure, Naphthalindisulfonsäure, Essigsaure, Propionsäure, Milchsäure, Weinsäure, Zitronensäure, Fumarsäure, Maleinsäure oder Benzoesäure.

Als Salze können weiterhin Salze mit üblichen Basen genannt werden, wie beispielsweise Alkalimetallsalze (z.B. Natrium- oder Kaliumsalze), Erdalkalisalze (z.B. Calcium- oder Magnesiumsalze) oder Ammoniumsalze, abgeleitet von Ammoniak oder organischen Aminen wie beispielsweise Diethylamin, Triethylamin, Ethyldiisopropylamin, Prokain, Dibenzylamin, N-Methylmorpholin, Dihydroabietylamin, 1-Ephenamin oder Methyl-piperidin.

Als Salze können außerdem Reaktionsprodukte mit $C_1$-$C_4$-Alkylhalogenide, insbesondere $C_1$-$C_4$-Alkyliodide fungieren.

Die erfindungsgemäßen Verbindungen können in stereoisomeren Formen, die sich entweder wie Bild und Spiegelbild (Enantiomere), oder die sich nicht wie Bild und Spiegelbild (Diastereomere) verhalten, existieren. Die Erfindung betrifft sowohl die Enantiomeren oder Diastereomeren oder deren jeweiligen Mischungen. Die Racemformen lassen sich ebenso wie die Diastereomeren in bekannter Weise in die stereoisomer einheitlichen Bestandteile trennen.

Bevorzugt sind Verbindungen der allgemeinen Formel (I),
in welcher

| | |
|---|---|
| A | für ein Sauerstoff- oder ein Schwefelatom steht, |
| D | für Wasserstoff, Cyclopropyl, Cyclopentyl oder Cyclohexyl steht, oder<br>für geradkettiges oder verzweigtes Alkoxycarbonyl mit bis zu 3 Kohlenstoffatomen steht, oder<br>für geradkettiges oder verzweigtes Alkyl oder Alkenyl mit jeweils bis zu 6 Kohlenstoffatomen steht, das gegebenenfalls durch Cyano, Trifluormethyl, Fluor, Chlor, Brom, Hydroxy, Pyridyl, Phenyl, Carboxyl, Carboxamido, geradkettiges oder verzweigtes Alkoxycarbonyl mit bis zu 4 Kohlenstoffatomen, Naphthyl, Cyclopropyl, Cyclopentyl oder Cyclohexyl und/oder durch eine Gruppe der Formel $-(CO)_a-NR^2R^3$, $R^4-N-SO_2-R^5$, $R^6R^7-N-SO_2-$ oder $R^8-S(O)_b$ substituiert ist,<br>worin |
| a | eine Zahl 0 oder 1 bedeutet, |
| $R^2$, $R^3$, $R^4$, $R^6$ und $R^7$ | gleich oder verschieden sind und Wasserstoff, geradkettiges oder ver- |

zweigtes Alkyl mit bis zu 3 Kohlenstoffatomen oder Phenyl bedeuten,
oder

| | |
|---|---|
| $R^2$ und $R^3$ | gemeinsam mit dem Stickstoffatom einen Morpholinyl-, Thiomorpholinyl-, Pyrrolidinyl-, Piperazinyl- oder Piperidylring bilden, die gegebenenfalls, auch über die freie N-Funktion, durch geradkettiges oder verzweigtes Alkyl oder Acyl mit jeweils bis zu 3 Kohlenstoffatomen substituiert sind, |
| b | eine Zahl 0, 1 oder 2 bedeutet, |
| $R^5$ und $R^8$ | gleich oder verschieden sind und geradkettiges oder verzweigtes Alkyl mit bis zu 4 Kohlenstoffatomen, Benzyl, Phenyl oder Tolyl bedeuten,<br>oder |
| D | für geradkettiges oder verzweigtes Acyl mit bis zu 4 Kohlenstoffatomen steht, das gegebenenfalls durch Trifluormethyl, Trichlormethyl oder eine Gruppe der Formel $-OR^9$ substituiert ist,<br>worin |
| $R^9$ | Wasserstoff oder geradkettiges oder verzweigtes Alkyl mit bis zu 4 Kohlenstoffatomen bedeutet, das gegebenenfalls durch Phenyl oder Naphthyl substituiert ist,<br>oder |
| D | für eine Gruppe der Formel $-(CT)_d-NR^{10}R^{11}$, $-(CO)_e-NR^{12}-CO-NR^{13}R^{14}$, $-NR^{15}-SO_2R^{16}$, $R^{17}R^{18}-N-SO_2-$, $R^{19}-S(O)_f$ oder $-CO-R^{20}$ steht,<br>worin |
| T | ein Sauerstoff- oder Schwefelatom bedeutet, |
| d und e | gleich oder verschieden sind und<br>die oben angegebene Bedeutung von a haben und mit dieser gleich oder verschieden sind, |
| $R^{10}$, $R^{11}$, $R^{12}$, $R^{13}$, $R^{14}$, $R^{15}$, $R^{17}$ und $R^{18}$ | die jeweils oben angegebene Bedeutung von $R^2$, $R^3$ und $R^4$ haben und mit dieser gleich oder verschieden sind, |
| f | die oben angegebene Bedeutung von b hat und mit dieser gleich oder verschieden ist, |
| $R^{16}$ und $R^{19}$ | gleich oder verschieden sind und die oben angegebene Bedeutung von $R^5$ und $R^8$ haben, |
| $R^{20}$ | Phenyl oder Naphthyl bedeutet, das gegebenenfalls durch Fluor, Chlor oder Brom substituiert ist, |
| E und L | gleich oder verschieden sind und<br>für Wasserstoff, Fluor, Chlor, Brom, Cyano, Methyl oder Trifluormethyl steht, |
| $R^1$ | für Azido, Hydroxy oder für eine Gruppe der Formel $-OR^{21}$, $O-SO_2R^{22}$ oder $-NR^{23}R^{24}$ steht,<br>worin |
| $R^{21}$ | geradkettiges oder verzweigtes Acyl mit bis zu 5 Kohlenstoffatomen oder Benzyl bedeutet, |
| $R^{22}$ | geradkettiges oder verzweigtes Alkyl mit bis zu 4 Kohlenstoffatomen, Phenyl oder Tolyl bedeutet, |

$R^{23}$ und $R^{24}$ gleich oder verschieden sind und Cyclopropyl, Cyclopentyl, Cyclohexyl, Wasserstoff, Phenyl oder geradkettiges oder verzweigtes Alkoxy mit bis zu 5 Kohlenstoffatomen, tert.Butoxycarbonyl oder Benzyloxycarbonyl bedeuten, oder geradkettiges oder verzweigtes Alkyl mit bis zu 4 Kohlenstoffatomen bedeuten, das gegebenenfalls durch Cyano oder durch geradkettiges oder verzweigtes Alkoxycarbonyl mit bis zu 3 Kohlenstoffatomen substituiert ist,
oder

$R^{23}$ und/oder $R^{24}$ eine Gruppe der Formel $-CT'-R^{25}$, $P(O)(OR^{26})(OR^{27})$ oder $-SO_2-R^{28}$ bedeutet,
worin

T die oben angegebene Bedeutung von T hat und mit dieser gleich oder verschieden ist,

$R^{25}$ Cyclopropyl, Cyclopentyl, Cyclohexyl, Trifluormethyl oder
geradkettiges oder verzweigtes Alkoxy mit bis zu 5 Kohlenstoffatomen, Phenyl, Benzyloxy oder Wasserstoff bedeutet, oder
geradkettiges oder verzweigtes Alkyl mit bis zu 5 Kohlenstoffatomen bedeutet, das gegebenenfalls durch geradkettiges oder verzweigtes Alkoxycarbonyl mit bis zu 4 Kohlenstoffatomen, Cyano, Fluor, Chlor, Brom oder Trifluormethyl substituiert ist, oder
geradkettiges oder verzweigtes Thioalkyl oder Acyl mit jeweils bis zu 4 Kohlenstoffatomen bedeutet, oder eine Gruppe der Formel $-NR^{29}R^{30}$ bedeutet,
worin

$R^{29}$ und $R^{30}$ gleich oder verschieden sind und Wasserstoff, Phenyl oder geradkettiges oder verzweigtes Alkyl mit bis zu 3 Kohlenstoffatomen bedeuten,

$R^{26}$ und $R^{27}$ gleich oder verschieden sind und Wasserstoff oder geradkettiges oder verzweigtes Alkyl mit bis zu 3 Kohlenstoffatomen bedeuten,

$R^{28}$ geradkettiges oder verzweigtes Alkyl mit bis zu 3 Kohlenstoffatomen oder Phenyl bedeutet,

und deren Salze und Isomere.

Besonders bevorzugt sind Verbindungen der allgemeinen Formel (I),
in welcher

A für ein Sauerstoff- oder ein Schwefelatom steht,

D für Wasserstoff, Cyclopropyl, Cyclopentyl, Cyclohexyl, Allyl oder für geradkettiges oder verzweigtes Alkyl mit bis zu 5 Kohlenstoffatomen steht, das gegebenenfalls durch Cyano, Hydroxy, Trifluormethyl, Fluor, Chlor, Phenyl, Carboxyl, Carboxamido, geradkettiges oder verzweigtes Alkoxycarbonyl mit bis zu 3 Kohlenstoffatomen, Cyclopropyl, Cyclopentyl oder Cyclohexyl und/oder durch eine Gruppe der Formel $-(CO)_a-NR^2R^3$, $R^4-N-SO_2-R^5$, $R^6R^7N-SO_2-$ oder $R^8-S(O)_b$ substituiert ist,
worin

a eine Zahl 0 oder 1 bedeutet,

$R^2$, $R^3$, $R^4$, $R^6$ und $R^7$ gleich oder verschieden sind und Wasserstoff oder Methyl bedeuten,

b eine Zahl 0, 1 oder 2 bedeutet,

$R^5$ und $R^8$ gleich oder verschieden sind und
geradkettiges oder verzweigtes Alkyl mit bis zu 3 Kohlenstoffatomen, Benzyl, Phenyl oder Tolyl bedeuten,
oder

D | für eine Gruppe der Formel $-(CT)_d-NR^{10}R^{11}$, $-(CO)_e-NR^{12}-CO-NR^{13}R^{14}$, $-NR^{15}-SO_2R^{16}$, $R^{17}R^{18}N-SO_2-$, $R^{19}-S(O)_f$ oder $-CO-R^{20}$ steht,
worin

T | ein Sauerstoff- oder Schwefelatom bedeutet,

d und e | gleich oder verschieden sind und
die oben angegebene Bedeutung von a haben und mit dieser gleich oder verschieden sind,

$R^{10}$, $R^{11}$, $R^{12}$, $R^{13}$, $R^{14}$, $R^{15}$, $R^{17}$ und $R^{18}$ | die jeweils oben angegebene Bedeutung von $R^2$, $R^3$ und $R^4$ haben und mit dieser gleich oder verschieden sind,

f | die oben angegebene Bedeutung von b hat und mit dieser gleich oder verschieden ist,

$R^{16}$ und $R^{19}$ | gleich oder verschieden sind und die oben angegebene Bedeutung von $R^5$ und $R^8$ haben,

$R^{20}$ | Phenyl oder Naphthyl bedeutet, das gegebenenfalls durch Fluor, Chlor oder Brom substituiert ist,

E und L | gleich oder verschieden sind und für Wasserstoff oder Fluor stehen,

$R^1$ | für Azido, Hydroxy oder für eine Gruppe der Formel $-OR^{21}$, $O-SO_2R^{22}$ oder $-NR^{23}R^{24}$ steht,
worin

$R^{21}$ | geradkettiges oder verzweigtes Acyl mit bis zu 4 Kohlenstoffatomen bedeutet,

$R^{22}$ | Methyl oder Toluolyl bedeutet,

$R^{23}$ und $R^{24}$ | gleich oder verschieden sind und
Cyclopropyl, Wasserstoff, Phenyl oder geradkettiges oder verzweigtes Alkoxy mit bis zu 4 Kohlenstoffatomen, tert.-Butoxycarbonyl oder Benzyloxycarbonyl bedeuten, oder
geradkettiges oder verzweigtes Alkyl mit bis zu 3 Kohlenstoffatomen bedeuten, das gegebenenfalls durch Cyano oder Methoxycarbonyl substituiert ist,
oder

$R^{23}$ und/oder $R^{24}$ | eine Gruppe der Formel $-CT'-R^{25}$ bedeutet,
worin

T' | die oben angegebene Bedeutung von T hat und mit dieser gleich oder verschieden ist,

$R^{25}$ | Cyclopropyl, Cyclopentyl, Cyclohexyl, Trifluormethyl oder
geradkettiges oder verzweigtes Alkoxy mit bis zu 4 Kohlenstoffatomen, Phenyl, Benzyloxy oder Wasserstoff bedeutet, oder
geradkettiges oder verzweigtes Alkyl mit bis zu 4 Kohlenstoffatomen bedeutet, das gegebenenfalls durch geradkettiges oder verzweigtes Alkoxycarbonyl mit bis zu 3 Kohlenstoffatomen, Cyano, Fluor, Chlor, Brom oder Trifluormethyl substituiert ist, oder
geradkettiges oder verzweigtes Thioalkyl oder Acyl mit jeweils bis zu 3 Kohlenstoffatomen bedeutet, oder eine Gruppe der Formel $-NR^{29}R^{30}$ bedeutet, worin

$R^{29}$ und $R^{30}$ gleich oder verschieden sind und Wasserstoff, Phenyl, Methyl oder Ethyl bedeuten,

und deren Salze und Isomere.

Ganz besonders bevorzugt sind Verbindungen der allgemeinen Formel (I), in welcher

E und L für Wasserstoff stehen und der Oxazolidinonrest in den Positionen 6 oder 7 an den 1,2-Dihydrochinolinylring angebunden ist.

Außerdem wurden Verfahren zur Herstellung der erfindungsgemäßen Verbindungen der allgemeinen Formel(I) gefunden, dadurch gekennzeichnet, daß man

[A] N-Oxide der allgemeinen Formel (II)

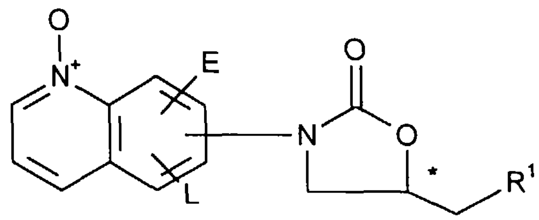

(II) ,

in welcher

E und L die oben angegebene Bedeutung haben und

$R^1$ die oben angegebene Bedeutung hat, vorzugsweise aber für den Rest der Formel $-NH-CO-NR^{31}$ steht, worin

$R^{31}$ geradkettiges oder verzweigtes Alkyl mit bis zu 4 Kohlenstoffatomen bedeutet,

durch Umsetzung mit $Ac_2O$ oder $p\text{-}TsCl/K_2CO_3$ in inerten Lösemitteln zunächst in die Verbindungen der allgemeinen Formel (Ia)

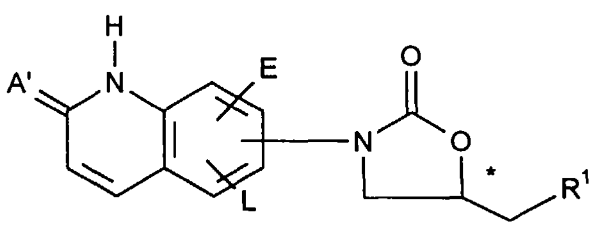

(Ia) ,

in welcher

$R^1$, E und L die oben angegebene Bedeutung haben und

A' für Sauerstoff steht,

überführt, oder

[B] Verbindungen der allgemeinen Formel (Ib)

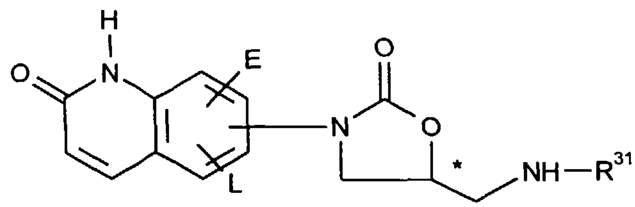

(Ib) ,

in welcher

E und L die oben angegebene Bedeutung haben

und $R^{31}$ für $C_1$-$C_4$-Acyl oder Alkoxycarbonyl steht,
mit Alkylierungsmitteln der allgemeinen Formel (III)

D'-X (III),

in welcher

D' die oben angegebene Bedeutung hat aber nicht für Wasserstoff steht und

X für Trifluormethansulfonat oder Halogen steht,

in inerten Lösemitteln und in Anwesenheit einer Base umsetzt,
und gegebenenfalls die anderen unter $R^1$ aufgeführten Substituenten nach üblichen Methoden einführt,
oder

[C] Verbindungen der allgemeinen Formel (IV)

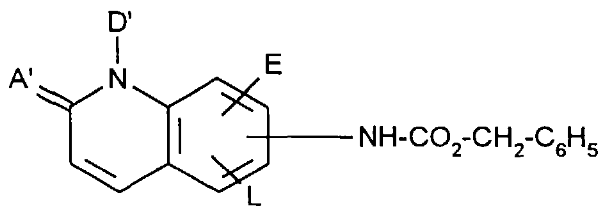

(IV) ,

in welcher

A', D', E und L die oben angegebene Bedeutung haben,

zunächst durch Umsetzung mit Lithiumalkylen in Ethern und R-Glycidylbutyrat der Formel (V)

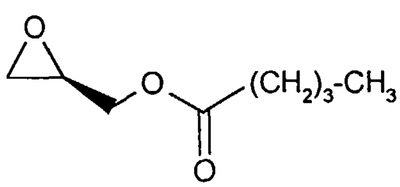

(V)

in die Verbindungen der allgemeinen Formel (Ic)

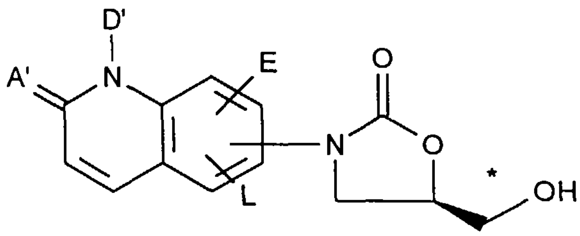

(Ic) ,

in welcher

A', D', E und L die oben angegebene Bedeutung haben,

überführt,
diese durch Umsetzung mit ($C_1$-$C_4$)-Alkyl- oder Phenylsulfonsäurechloriden in inerten Lösemitteln und in Anwesenheit einer Base in die entsprechenden Verbindungen der allgemeinen Formel (Id)

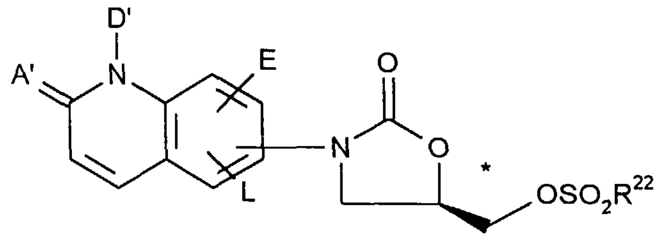

(Id) ,

in welcher

A', D', E, L und $R^{22}$ die oben angegebene Bedeutung haben,

überführt,
anschließend mit Natriumazid in inerten Lösemitteln die Azide der allgemeinen Formel (Ie)

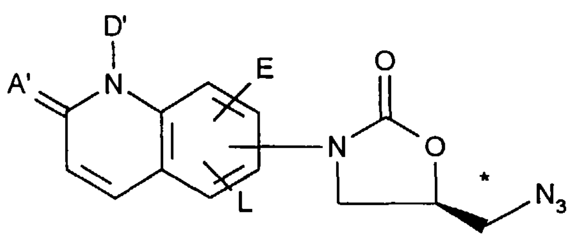

(Ie) ,

in welcher

A', D', E und L die oben angegebene Bedeutung haben,

herstellt,
diese in einem weiteren Schritt durch Umsetzung mit Alkylphosphiten oder $PPh_3$, vorzugsweise $(CH_3O)_3P$ in inerten Lösemitteln und mit Säuren in die Amine der allgemeinen Formel (If)

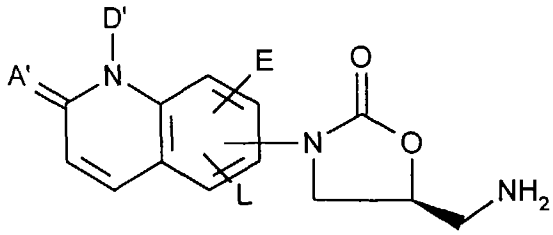

(If) ,

in welcher

A', D', E und L die oben angegebene Bedeutung haben,

überführt,
und durch Umsetzung mit Acetanhydrid oder anderen Acylierungsmitteln der allgemeinen Formel (VI)

$$R^{32}\text{-CT'-}R^{25} \qquad \text{(VI)},$$

in welcher

$R^{25}$ und T' die angegebene Bedeutung haben und

$R^{32}$ für Halogen, vorzugsweise für Chlor oder für den Rest $-OCOR^{25}$ steht,

in inerten Lösemitteln die Verbindungen der allgemeinen Formel (Ig)

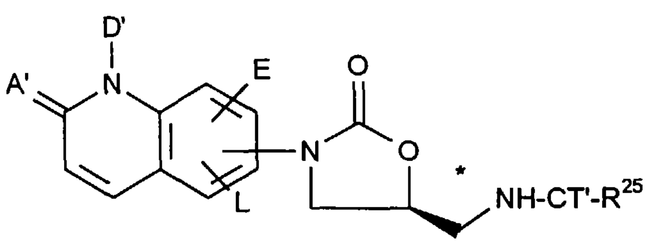

(Ig) ,

in welcher

A', D', E, L, T' und $R^{25}$ die oben angegebene Bedeutung haben

herstellt,
und im Fall A = S beispielsweise Verbindungen der allgemeinen Formel (Ig) einer Schwefelung der Amidfunktion mit Lawessons-Reagenz oder $P_2S_5$ in Toluol oder 1,2-Dimethoxyethan unterzieht.

Die erfindungsgemäßen Verfahrensvarianten können durch das folgende Formelschema beispielhaft erläutert werden:

[A]

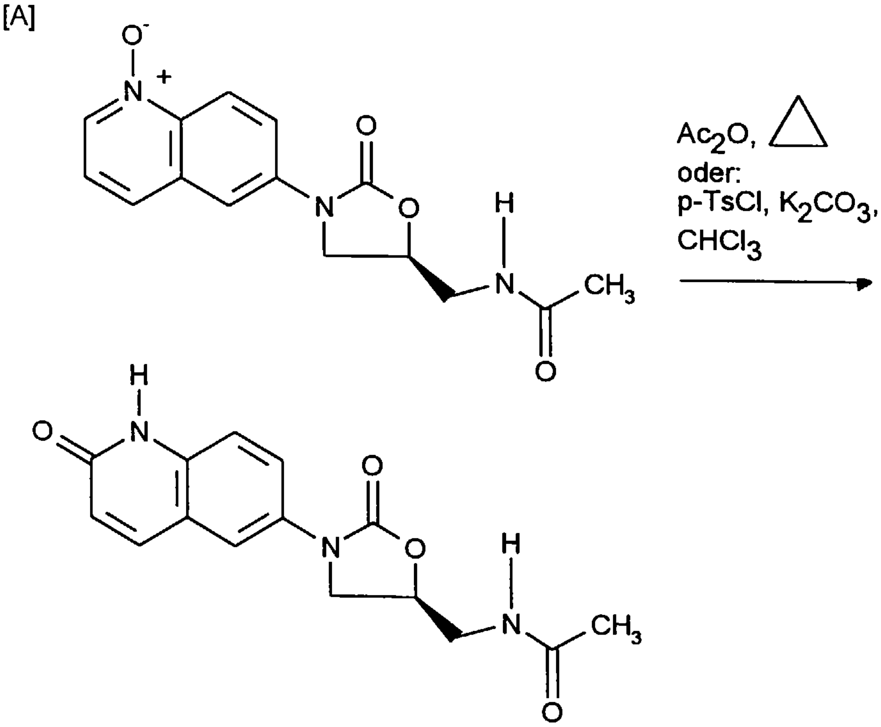


[B]

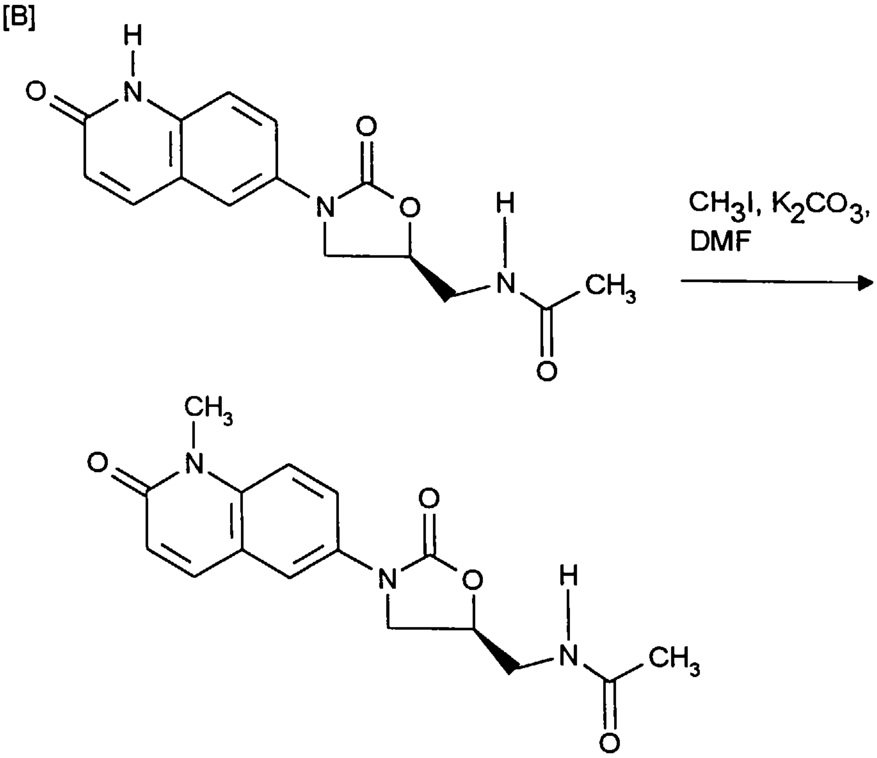

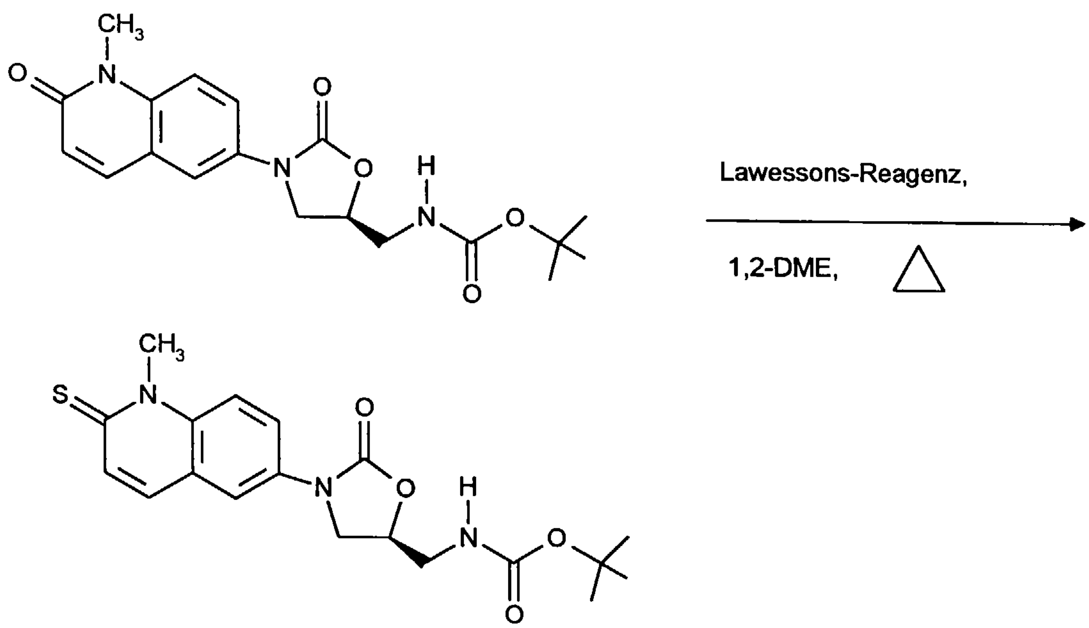
Lawessons-Reagenz,
1,2-DME,

[C]

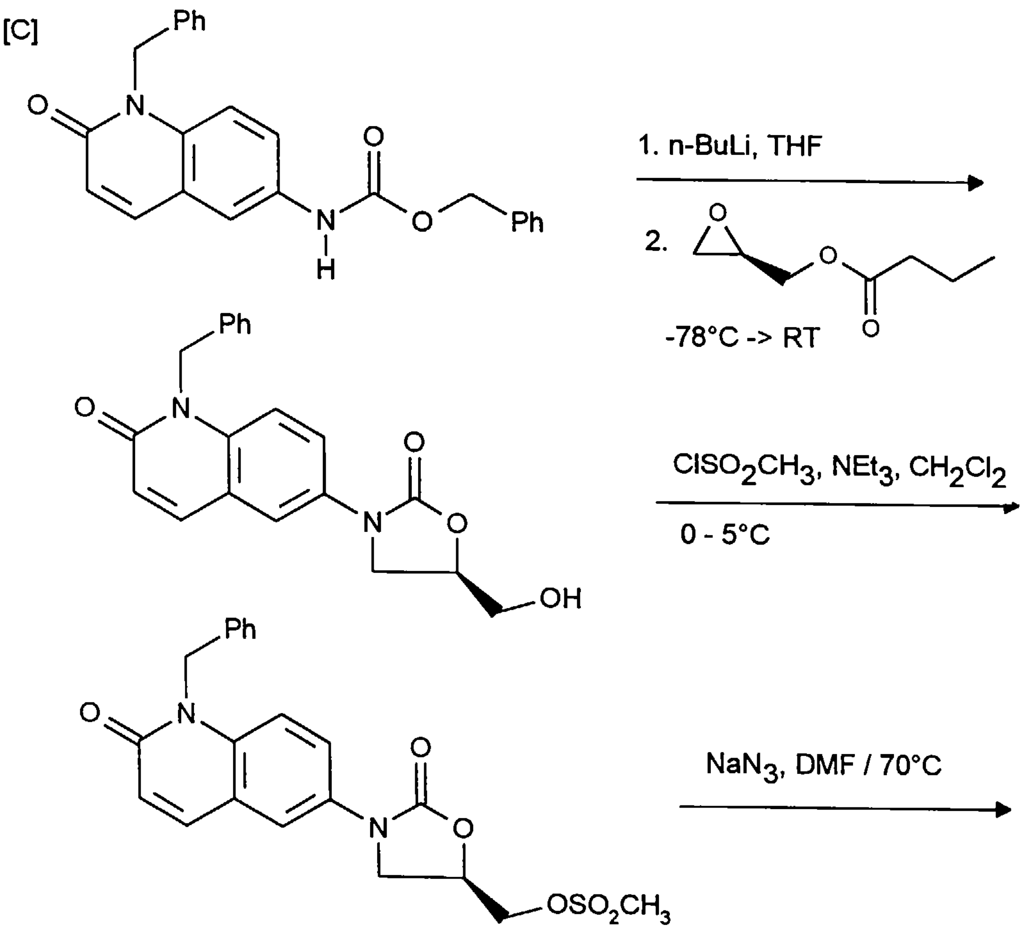
1. n-BuLi, THF
2.
-78°C -> RT
ClSO2CH3, NEt3, CH2Cl2
0 - 5°C
NaN3, DMF / 70°C

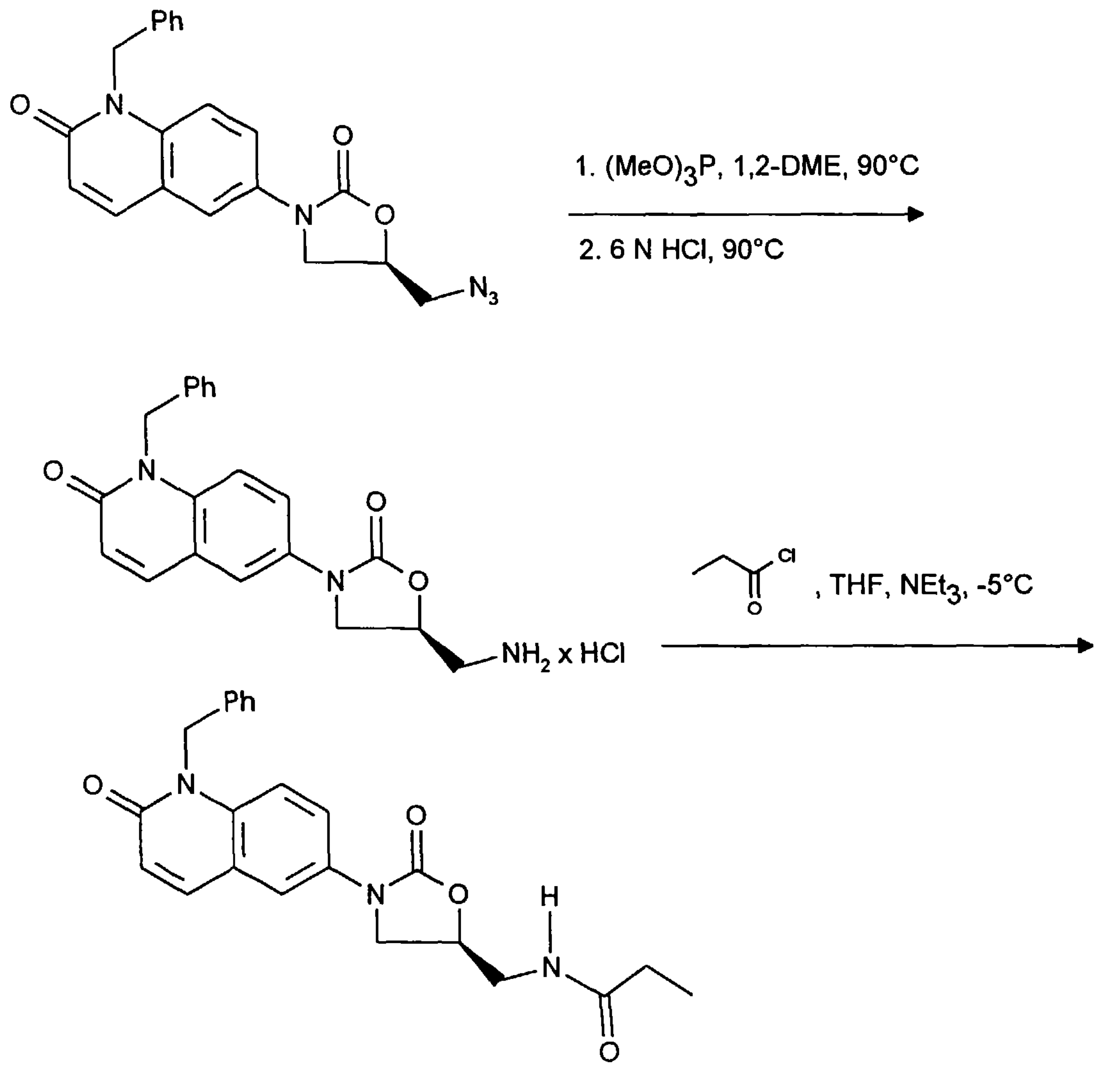


Als Lösemittel eignen sich in Abhängigkeit von den einzelnen Verfahrensschritten die üblichen Lösemittel, die sich unter den Reaktionsbedingungen nicht verändern. Hierzu gehören bevorzugt Alkohole wie Methanol, Ethanol, Propanol oder Isopropanol, oder Ether wie Diethylether, Dioxan, 1,2-Dimethoxyethan, Tetrahydrofuran, Glykoldimethylether oder tert.Butylmethylether, oder Ketone wie Aceton oder Butanon, oder Amide wie Dimethylformamid oder Hexamethyl-phosphorsäuretriamid, oder Kohlenwasserstoffe wie Hexan, Benzol, Dichlorbenzol, Xylol oder Toluol, oder Dimethylsulfoxid, Acetonitril, Essigester, oder Halogenkohlenwasserstoffe wie Methylenchlorid, Chloroform oder Tetrachlorkohlenstoff, oder Pyridin, Picolin oder N-Methylpiperidin. Ebenso können Gemische der genannten Lösemittel verwendet werden.

Als Basen eignen sich in Abhängigkeit von den einzelnen Verfahrensschritten die üblichen anorganischen oder organischen Basen. Hierzu gehören bevorzugt Alkalihydroxide wie beispielsweise Natrium- oder Kaliumhydroxid, oder Alkalicarbonate wie Natrium- oder Kaliumcarbonat, oder Alkalialkoholate wie beispielsweise Natrium- oder Kaliummethanolat, oder Natrium- oder Kaliumethanolat, oder organische Amine wie Ethyldiisopropylamin, Triethylamin, Picolin, Pyridine oder N-Methylpiperidin, oder Amide wie Natriumamid oder Lithiumdiisopropylamid, oder Lithium-N-silylalkylamide, wie beispielsweise Lithium-N-(bis)triphenysilylamid oder Lithiumalkyle wie n-Butyllithium.

Die Base wird in einer Menge von 1 mol bis 10 mol, bevorzugt von 1 mol bis 3 mol bezogen auf 1mol der Verbindungen der allgemeinen Formeln (Ib), (II), (III) bzw. (IV) eingesetzt.

Alle Umsetzungen werden im allgemeinen bei normalem, erhöhtem oder bei erniedrigtem Druck durchgeführt (z.B. 0,5 bis 5 bar). Im allgemeinen arbeitet man bei Normaldruck.

Als Lösemittel für die Alkylierung eignen sich übliche organische Lösemittel, die sich unter den Reaktionsbedingungen nicht verändern. Hierzu gehören bevorzugt Ether wie Diethylether, Dioxan, Tetrahydrofuran, Glykoldimehylether, oder Kohlenwasserstoffe wie Benzol, Toluol, Xylol, Hexan, Cyclohexan oder Erdölfraktionen, oder Halogenkohlenwasserstoffe wie Dichlormethan, Trichlormethan, Tetrachlormethan, Dichlorethylen, Trichlorethylen oder Chlorbenzol, oder Essigester, oder Triethylamin, Pyridin, Dimethylsulfoxid, Dimethylformamid, Acetonitril, Aceton oder Nitromethan. Ebenso ist es möglich, Gemische der genannten Lösemittel zu verwenden. Bevorzugt sind Acetonitril, Dimethylsulfoxid und Dimethylformamid.

Die Alkylierung wird in den oben aufgeführten Lösemitteln bei Temperaturen von 0°C bis +150°C, vorzugsweise bei Raumtemperatur bis +100°C, bei Normaldruck durchgeführt.

Der erste Schritt des Verfahrens [B] erfolgt in einem der oben aufgeführten Ether mit Lithiumalkylverbindungen oder Lithium-N-silylamiden, wie beispielsweise n-Butyllithium, Lithiumdiisopropylamid oder Lithium-bistrimethylsilylamid, vorzugsweise in Tetrahydrofuran und Lithium-bis-trimethylsilylamid oder n-Butyllithium, in einem Temperaturbereich von -100°C bis +20°C, vorzugsweise von -75°C bis -40°C.

Im weiteren werden für die einzelnen Schritte die oben aufgeführten Lösemittel, vorzugsweise Methylenchlorid, Dimethylformamid und 1,2-Dimethoxyethan eingesetzt.

Die Acylierung erfolgt im allgemeinen in einem der oben aufgeführten Ether oder Halogenkohlenwasserstoffen, vorzugsweise Tetrahydrofuran oder Methylenchlorid, in einem Temperaturbereich von -30°C bis 50°C, bevorzugt von -10°C bis Raumtemperatur.

Die Verbindungen der allgemeinen Formel (II) sind neu und können hergestellt werden, indem man Verbindungen der allgemeinen Formel (VII)

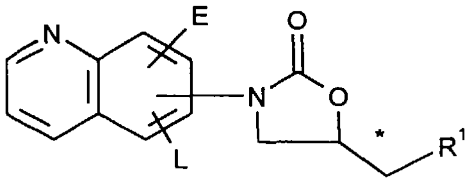

(VII) ,

in welcher

$R^1$, E und L die oben angegebene Bedeutung haben,

in einem der oben aufgeführten Lösemittel, vorzugsweise in Methylenchlorid mit Oxidationsmitteln wie beispielsweise Metachlorperbenzoesäure, Wasserstoffperoxid oder Peressigsaure, vorzugsweise mit Metachlorperbenzoesäure in einem Temperaturbereich von 0°C bis 80°C, bevorzugt von 0°C bis 40°C, umsetzt.

Die Verbindungen der allgemeinen Formel (VII) können hergestellt werden, indem man in Analogie zu dem oben aufgeführten Verfahren [C] Verbindungen der allgemeinen Formel (VIII)

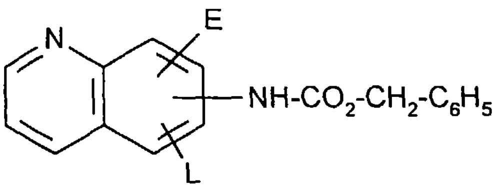

(VIII) ,

in welcher

E und L die oben angegebene Bedeutung haben,

mit dem Epoxid der Formel (IV) umsetzt und ausgehend von der freien Hydroxyfunktion ebenfalls in Analogie zu den oben aufgeführten Verbindungen die Gruppe $R^1$ einführt.

Die Verbindungen der allgemeinen Formeln (III), (V), (VI) und (VIII) sind an sich bekannt oder nach üblichen Methoden herstellbar.

Die Verbindungen der allgemeinen Formel (IV) sind neu und können hergestellt werden, indem man in bekannten Aminen der allgemeinen Formel (IX)

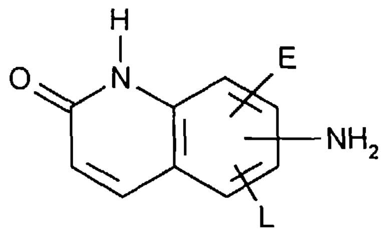

(IX) ,

in welcher

E und L die oben angegebene Bedeutung haben,

zunächst die freie Aminofunktion mit $ClCO_2CH_2C_6H_5$ in Tetrahydrofuran bei pH 10 schützt und in einem zweiten Schritt eine Alkylierung (D' ≠ H) nach den oben aufgeführten Bedingungen durchführt.

Die Blockierung der freien Aminfunktion erfolgt in einem Temperaturbereich von 150°C bis 200°C, vorzugsweise bei 180°C und Normaldruck.

Die Verbindungen der allgemeinen Formeln (Ia) - (Ig) sind neu und können jeweils wie oben beschrieben hergestellt werden.

Die minimalen Hemmkonzentrationen (MHK) wurden per Reihenverdünnungsverfahren auf Iso-Sensitest Agar (Oxoid) bestimmt. Für jede Prüfungssubstanz wurde eine Reihe von Agarplatten hergestellt, die bei jeweils doppelter Verdünnung abfallende Konzentrationen des Wirkstoffes enthielten. Die Agarplatten wurden mit einem Multipoint-Inokulator (Denley) beimpft. Zum Beimpfen wurden Übernachtkulturen der Erreger verwandt, die zuvor so verdünnt wurden, daß jeder Impfpunkt ca. $10^4$ koloniebildende Partikel enthielt. Die beimpften Agarplatten wurden bei 37°C bebrütet, und das Keimwachstum wurde nach ca. 20 Stunden abgelesen. Der MHK-Wert (µg/ml) gibt die niedrigste Wirkstoffkonzentration an, bei der mit bloßem Auge kein Wachstum zu erkennen war.

| **MHK-Werte (µg/ml):** | | | | | | | |
|---|---|---|---|---|---|---|---|
| **Bsp. -Nr.** | **Staph. 133** | **Staph. 48N** | **Staph 25701** | **Staph. 9TV** | **E. coli Neumann** | **Klebs. 57 USA** | **Psdm. Bonn** |
| 3 | 2 | 2 | 2 | 1 | >64 | >64 | >64 |
| 4 | 4 | 8 | 4 | 2 | >64 | >64 | - |
| 5 | 16 | 16 | 16 | 8 | >64 | >64 | >64 |
| 7 | 8 | 8 | 8 | 2 | >32 | >32 | >32 |
| 12 | 8 | 8 | 8 | 4 | >64 | >64 | >64 |

Für schnellwachsende Mykobakterien wurde die MHK-Bestimmung in Anlehnung an die von Swenson beschriebene Methode der Bouillon-Mikrodilution durchgeführt [vgl. J.M. Swenson, C. Thornberry, U.A. Silcox, Rapidly growing mycobacteria. Testing of susceptibility to 34 antimicrobial agents by broth microdilution. Antimicrobial Agents and Chemotherapy Vol, 22, 186-192 (1982)]. Abweichend davon war das mit 0,1 Vol.% Tween 80 versetzte Hirn-Herzextrakt Medium.

Die verwendeten Mykobakterienstämme wurden von der DSM (Dt. Sammlung von Mikroorganismen, Braunschweig) bezogen. Sie wurden in einer feuchten Kammer bei 37°C bebrütet.

Die MHK-Werte wurden nach 2-4 Tagen abgelesen, wenn die präparatfreien Kontrollen durch Wachstum trüb waren. Der MHK-Wert definiert sich als die niedrigste Präparatkonzentration, die makroskopisch sichtbares Wachstum völlig inhibiert.

| MHK-Werte: Mycobacterium smegmatis | | |
|---|---|---|
| **Stamm:** | **DSM 43061** | **DSM 43465** |
| Inoculum [/ml] | 2,20E+04 | 3,10E+04 |
| Bsp.-Nr. | | |
| 3 | 2 | 8 |
| 4 | 4 | 8 |
| 6 | 8 | 4 |
| Isoniazid | 4 | 1 |
| Streptomycin | 4 | 4 |

Die erfindungsgemäßen Verbindungen der allgemeinen Formeln (I), (Ia), (Ib), (Ic), (Id), (Ie), (If) und (Ig) weisen bei geringer Toxizität ein breites antibakterielles Spektrum, speziell gegen gram-positive Bakterien sowie Mycobacterien, Haemophilus influenzae, anaerobe Keime und für schnellwachsende Mykobakterien auf. Diese Eigenschaften ermöglichen ihre Verwendung als chemotherapeutische Wirkstoffe in der Human- und Tiermedizin.

Besonders wirksam sind die erfindungsgemäßen Verbindungen gegen Bakterien und bakterienähnliche Mikroorganismen wie Mycoplasmen. Sie sind daher besonders gut zur Prophylaxe und Chemotherapie von lokalen und systemischen Infektionen in der Human- und Tiermedizin geeignet, die durch solche Erreger hervorgerufen werden.

Zur vorliegenden Erfindung gehören pharmazeutische Zubereitungen, die neben nicht-toxischen, inerten pharmazeutisch geeigneten Trägerstoffen eine oder mehrere erfindungsgemäße Verbindungen enthalten oder die aus einem oder mehreren erfindungsgemäßen Wirkstoffen bestehen, sowie Verfahren zur Herstellung dieser Zubereitungen.

Der oder die Wirkstoffe können gegebenenfalls in einem oder mehreren der oben angegebenen Trägerstoffe auch in mikroverkapselter Form vorliegen.

Die therapeutisch wirksamen Verbindungen sollen in den oben aufgeführten pharmazeutischen Zubereitungen in einer Konzentration von etwa 0,1 bis 99,5, vorzugsweise von etwa 0,5 bis 95 Gew.-%, der Gesamtmischung vorhanden sein.

Die oben aufgeführten pharmazeutischen Zubereitungen können außer den erfindungsgemäßen Verbindungen auch weitere pharmazeutische Wirkstoffe enthalten.

Im allgemeinen hat es sich sowohl in der Human- als auch in der Veterinärmedizin als vorteilhaft erwiesen, den oder die erfindungsgemäßen Wirkstoffe in Gesamtmengen von etwa 0,5 bis etwa 500, vorzugsweise 5 bis 100 mg/kg Körpergewicht je 24 Stunden, gegebenenfalls in Form mehrerer Einzelgaben, zur Erzielung der gewünschten Ergebnisse zu verabreichen. Eine Einzelgabe enthält den oder die erfindungsgemäßen Wirkstoffe vorzugsweise in Mengen von etwa 1 bis etwa 80, insbesondere 3 bis 30mg/kg Körpergewicht.

Die erfindungsgemäßen Verbindungen können zum Zweck der Erweiterung des Wirkungsspektrums und um eine Wirkungssteigerung zu erreichen auch mit anderen Antibiotika kombiniert werden.

Anhang zum experimentellen Teil

Abkürzungen:

| | |
|---|---|
| Z | Benzyloxycarbonyl |
| Boc | tert.Butyloxycarbonyl |
| DMF | Dimethylformamid |
| Ph | Phenyl |
| Me | Methyl |
| THF | Tetrahydrofuran |
| CDI | Carbonyldiimidazol |
| DCE | Dichlorethan |

## Ausgangsverbindungen

### Beispiel I

6-Benzyloxycarbonylamino-chinolin

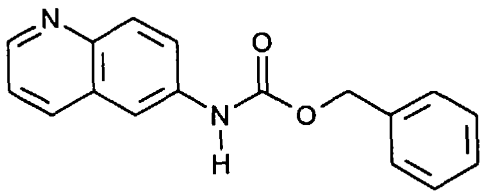

Zu einer auf 0°C gekühlten, gerührten Lösung von 10,0 g (69,36 mmol) 6-Aminochinolin in 160 ml Wasser und 80 ml THF werden innerhalb von 30 min 13,0 ml (76,28 mmol) Chlorameisensäurebenzylester getropft, wobei pH = 10 durch gleichzeitige Zugabe einer 4 N NaOH Lösung gehalten wird. Man rührt noch 2 h bei 0°C nach, dampft das THF im Vakuum ab und extrahiert den Rückstand mit 3 x 50 ml Ethylacetat. Die vereinigten organischen Extrakte werden über $MgSO_4$ getrocknet, das Lösemittel wird im Vakuum abgedampft und der Rückstand durch Chromatographie an 450 g Kieselgel (Toluol : Ethylacetat 1:4) gereinigt. Man erhält 11,60 g (60%) der Titelverbindung als Kristalle.

Schmp.: 122°C
$R_f$ = 0,43 (Toluol : Ethylacetat 1:4)
MS (EI) m/z = 278 ($M^+$)
$^1$H-NMR (300 MHz, $D_6$-DMSO): δ = 5,22 (s, 2H, $CH_2O$); 7,3 - 7,5 (m, 6H, Ph, Chinolin-H); 7,78 (dd, J = 1,5, 9 Hz, 1H, Chinolin-H); 7,96 (d, J = 9 Hz, 1H, Chinolin-H); 8,17 (d, J = 1,5 Hz, 1H, Chinolin H-5); 8,25 (d, J = 9 Hz, 1H, Chinolin-H); 8,77 (m, 1H, Chinolin H-2).

### Beispiel II

(5R)-3-(Chinolin-6-yl)-5-hydroxymethyl-oxazolidin-2-on

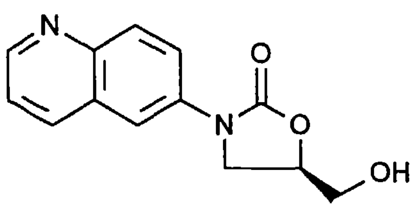

Eine auf -78°C gekühlte, gerührte Lösung von 3,28 g (11,78 mmol) 6-Benzyloxycarbonylamino-chinolin und 1 mg 1,10-Phenanthrolinhydrat in 30 ml wasserfreiem THF wird bis zum Farbumschlag langsam mit 4,70 ml (11,78 mmol) einer 2,5 M Lösung von n-Butyllithium in n-Hexan versetzt. Danach tropft man 1,67 ml (11,78 mmol) (R)-Glycidylbutyrat zu und erlaubt der Reaktionsmischung sich innerhalb von 16 h auf Raumtemperatur zu erwärmen. Danach werden innerhalb von 15 min 30 ml gesättigte wässrige $NH_4Cl$-Lösung zugetropft. Die Wasserphase wird mit 3 x 60 ml Ethylacetat extrahiert, die organischen Phasen werden vereinigt, mit 2 x 50 ml NaCl-Lösung gewaschen und über $MgSO_4$ getrocknet. Nach Abdampfen des Lösemittels im Vakuum, Verreiben des Rückstands mit Ether und Umkristallisation aus 25 ml Ethanol erhält man 1,30 g (45%) der Titelverbindung als farblose Kristalle.

Schmp.: 165°C
$R_f$ = 0,08 (Toluol : Ethylacetat 1:4)
MS (DCI, $NH_3$) m/z = 245 $(M+H)^+$
$^1$H-NMR (250 MHz, $D_6$-DMSO) δ = 3,6 - 3,8 (m, 2H, $CH_2O$); 4,00 (dd, J = 7, 10 Hz, 1H, H-4 trans); 4,25 (dd, J = 10, 10 Hz, 1H, H-4 cis); 4,78 (m, 1H, H-5); 5,25 (t, J = 6 Hz, 1H, OH); 7,52 (dd, J = 4, 9 Hz, 1H, Chinolin H-3); 7,92 (d, J = 1,5 Hz, 1H, Chinolin H-5); 8,02 (d, J = 10 Hz, 1H, Chinolin H-8); 8,3 (m, 2H, Chinolin H-4,7); 8,82 (m, 1H,

Chinolin H-2).

**Beispiel III**

(5R)-3-(5-Chinolin-6-yl)-5-methansulfonyloxy-methyl-oxazolidin-2-on

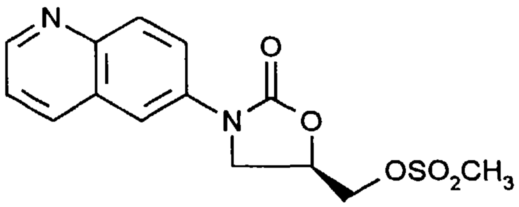

Eine auf 0°C gekühlte, gerührte Lösung von 48,19 g (197 mmol) der Verbindung aus Beispiel II und 33 ml (236 mmol) Triethylamin in 300 ml wasserfreiem Dichlormethan wird langsam mit 19,80 ml (256 mmol) Methansulfonsäurechlorid versetzt. Man rührt 10 min. bei 0-5°C nach und rührt das Gemisch in 700 ml Eiswasser ein. Die organische Phase wird abgetrennt, mit 100 ml gesättigter $NaHCO_3$-Lösung und 100 ml Eiswasser gewaschen und über $MgSO_4$ getrocknet. Das Lösemittel wird im Vakuum eingedampft und der Rückstand mit 50 ml Ether verrührt, abgesaugt und im Hochvakuum getrocknet. Man erhält 46,0 g (72%) der Titelverbindung als farblose Kristalle.

Schmp.: 143°C
$R_f$ = 0,14 (Toluol : Ethylacetat 1:9)
MS (DCI, $NH_3$) m/z = 323 $(M+H)^+$
$^1$H-NMR (200 MHz, $D_6$-DMSO) $\delta$ = 3,27 (s, 3H, $OSO_2CH_3$); 4,00 (dd, J = 7, 10 Hz, 1H, H-4 trans); 4,36 (dd, J = 10, 10 Hz, 1H, H-4 cis); 4,57 (m, 2H, CH $_2$O); 5,10 (m, 1H, H-5); 7,53 (dd, J = 4, 9 Hz, 1H, Chinolin H-3); 7,93 (d, J = 1,5 Hz, 1H, Chinolin H-5); 8.06 (d, J = 10 Hz, 1H, Chinolin H-8); 8,25 (dd, J = 1,5, 10 Hz, 1H, Chinolin H-7); 8,36 (d, J = 9 Hz, 1H, Chinolin H-4); 8,84 (m, 1H, Chinolin H-2).

**Beispiel IV**

(5R)-3-(Chinolin-6-yl)-5-azidomethyl-oxazolidin-2-on

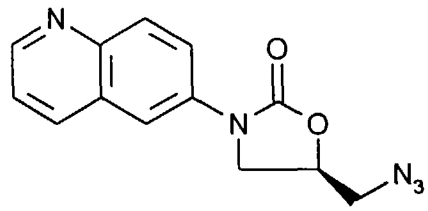

Eine gerührte Lösung von 4,71 g (14,60 mmol) der Verbindung aus Beispiel III in 16 ml wasserfreiem DMF wird mit 1,14 g (17,52 mmol) Natriumazid versetzt und 3 h bei 70°C gerührt. Man läßt auf Raumtemperatur abkühlen und rührt in 50 ml Eiswasser ein. Der entstandene Niederschlag wird durch Filtration abgetrennt, dreimal mit 20 ml Wasser gewaschen und an der Luft getrocknet. Man erhält 3,50 g (89%) der Titelverbindung als helle Kristalle.

Schmp.: ab 92°C
$R_f$ = 0,20 (Toluol : Ethylacetat 1:9)
MS (DCI, $NH_3$) m/z = 270 $(M+H)^+$
$^1$H-NMR (250 MHz, $D_6$-DMSO) $\delta$ = 3,71 (m, 2H, $CH_2N_3$); 3,95 (dd, J = 6, 8 Hz, 1H, H-4 trans); 4,30 (dd, J = 8, 8 Hz, 1H, H-4 cis); 4,98 (m, 1H, H-5); 7,52 (dd, J = 9 Hz, 1H, Chinolin H-3); 7,94 (d, J = 1,5 Hz, 1H, Chinolin H-5); 8,06 (d, J = 10 Hz, 1H, Chinolin H-8); 8,25 (dd, J = 1,5 Hz, 10 Hz, 1H, Chinolin H-7); 8,34 (d, J = 9 Hz, 1H, Chinolin

H-4); 8,84 (m, 1H, Chinolin H-2).

**Beispiel V**

(5S)-3-(Chinolin-6-yl)-5-aminomethyl-oxazolidin-2-on Dihydrochlorid

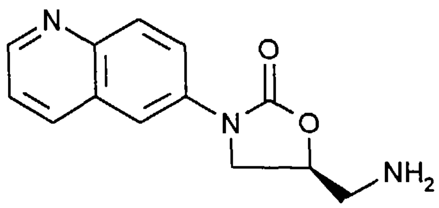

x 2 HCl

Eine gerührte Lösung von 29,38 g (109 mmol) der Verbindung aus Beispiel IV in 80 ml 1,2-Dimethoxyethan wird auf 50°C erwärmt. Man tropft langsam 25 ml (130 mmol) Trimethylphosphit zu (Gasentwicklung) und rührt nach beendeter Zugabe noch 2 h bei 90°C nach. Nun tropft man 3,3 ml 6 N HCl zu und rührt nochmals 6,5 h bei 100°C nach. Man läßt auf Raumtemperatur abkühlen und trennt das entstandene Öl ab. Das Öl wird in wenig Acetonitril gelöst, mit 50 ml Toluol versetzt, im Vakuum eingeengt und im Hochvakuum über NaOH getrocknet. Man erhält 30,5 g (99%) der Titelverbindung. Der Hartschaum wird aus Ethanol umkristallisiert.

Schmp.: 80°C (Zers.)
$R_f$ = 0,37 (Acetonitril : Wasser 4:1)
MS (FAB) m/z = 244 $(M+H)^+$
$^1$H-NMR (250 MHz, $D_6$-DMSO): δ = 3,35 (m, 2H, $\underline{CH_2}NH_2$); 4,08 (dd, J = 7, 9 Hz, 1H, H-4 trans); 4,39 (dd, J = 9, 9 Hz, 1H, H-4 cis); 5,10 (m, 1H, H-5); 7,90 (dd, J = 4, 9 Hz, 1H, Chinolin H-3); 8,17 (d, J = 1,5 Hz, 1H, Chinolin H-5); 8,3 - 8,6 (m, 3H, Chinolin H-8, 7,5); 8,90 (d, J = 9 Hz, 1H, Chinolin H-4); 9,10 (m, 1H, Chinolin H-2).

**Beispiel VI**

(5S)-3-(Chinolin-6-yl)-5-acetylaminomethyl-oxazdidin-2-on

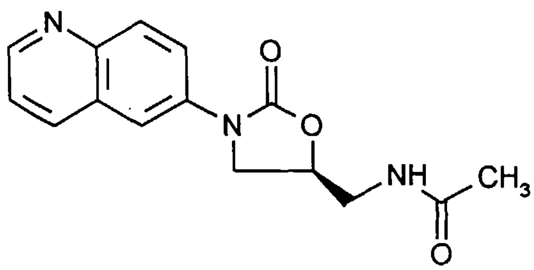

Eine gerührte Lösung von 30,0 g (108 mmol) der Verbindung aus Beispiel V in 240 ml THF wird mit einer Lösung von 10 g Natriumhydroxid in 20 ml Wasser versetzt, wobei pH 7,1 entsteht. Dazu tropft man bei 0-5°C langsam 11,6 ml (120 mmol) Acetanhydrid in 12 ml THF und hält pH = 9 durch gleichzeitige Zugabe einer 5 N wäßrigen NaOH-Lösung. Man rührt 1 h bei 0°C nach und dampft das Lösemittel im Vakuum ab. Der Rückstand wird mit 2 x 40 ml Wasser gut verrührt, abgetrennt und im Hochvakuum über Sicapent getrocknet. Man erhält 19,16 g (62%) der Titelverbindung als farblose Kristalle.

Schmp.: 146°C
$R_f$ = 0,33 (Dichlormethan : Methanol 9:1)
MS (FAB) m/z = 286 $(M+H)^+$
$^1$H-NMR (250 MHz, $D_6$-DMSO) δ = 1,85 (s, 3H, $COCH_3$); 3,50 (t, J = 6,5 Hz, 2H, $CH_2N$); 3,90 (dd, J = 7, 9 HZ, 1H, H-4 trans); 4,28 (dd, J = 9, 10 Hz, 1H, H-4 cis); 4,80 (m, 1H, H-5); 7,52 (dd, J = 4, 9 Hz, 1H, Chinolin H-3); 7,88 (d,

J = 1,5 Hz, 1H, Chinolin H-5); 8,05 (d, J = 10 Hz, 1H, Chinolin H-8); 8,25 (dd, J = 1-5, 10 Hz, Chinolin H-7); 8,34 (d, J = 9 Hz, 1H, Chinolin H-4); 8,82 (m, 1H, Chinolin H-2).

**Beispiel VII**

(5R)-3-(Chinolin-6-yl)-5-acetylaminomethyl-oxazolidin-2-on-N-1-oxid

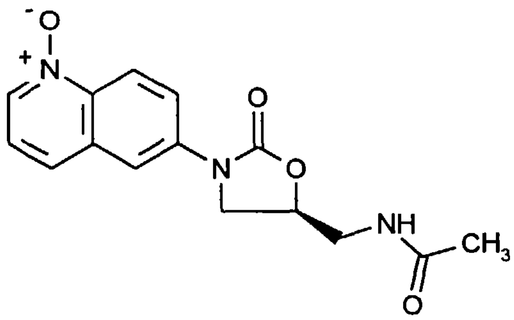

Eine gerührte Lösung von 500 mg (1,75 mmol) der Verbindung aus Beispiel VI in 5 ml Dichlormethan wird mit 832 mg (3,85 mmol) 80%iger m-Chlorperbenzosäure versetzt und 16 h bei Raumtemperatur gerührt. Danach wird die Reaktionsmischung in 20 ml gesättigter wäßrige $Na_2CO_3$-Lösung eingerührt. Die wäßrige Phase wird abgetrennt und im Vakuum eingedampft. Man gibt 25 ml Toluol und 1,5 g Kieselgel zu und dampft erneut ein. Der Rückstand wird durch Chromatographie an 50 g Kieselgel (Dichlormethan : Methanol 4:1) gereinigt. Die produkthaltigen Fraktionen werden vereinigt und mit 200 ml Ether versetzt. Der entstandene Niederschlag wird durch Filtration abgetrennt und im Hochvakuum getrocknet. Man erhält 453 mg (86%) der Titelverbindung als farblose Kristalle.

Schmp.: 191°C (Zers.)
$R_f$ = 0,15 (Dichlormethan : Methanol 9:1)
MS (FAB) m/z = 302 $(M+H)^+$
$^1$H-NMR (300 MHz, $D_6$-DMSO): $\delta$ = 1,85 (s, 3H, $COCH_3$); 3,50 (m, 2H, $CH_2N$); 3,91 (dd, J = 7, 10 Hz, 1H, H-4 trans); 4,28 (dd, J = 10, 10 Hz, 1H, H-4 cis); 4,82 (m, 1H, H-5); 7,3 - 7,5 (m, 2H); 7,9 (m, 1H); 8,0 (s, 1H, Chinolin H-5); 8,3 (m, 1H); 8,50 (m, 1H, Chinolin H-2).

## Herstellungsbeispiele

### Beispiel 1

(5S)-3-(2-Oxo-1,2-dihydro-chinolin-6-yl)-5-acetylaminomethyl-oxazolidin-2-on

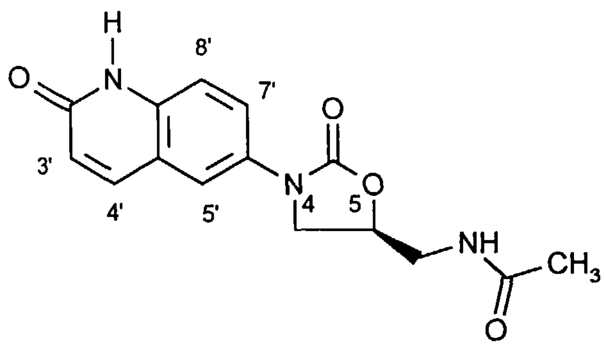

Methode A:

Eine Suspension von 2,73 g (9,10 mmol) des N-Oxids aus Beispiels VII und 2,59 g (13,59 mmol) p-Toluolsulfonsäurechlorid in 35 ml Chloroform wird mit 27,20 ml (27,20 mmol) einer 10%igen wäßrigen Kaliumcarbonatlösung versetzt und 4 h bei Raumtemperatur gut durchgerührt. Danach wird das Reaktionsgemisch zur Trockene eingedampft und an 100 g Kieselgel (Acetonitril : Wasser 95:5) chromatographiert. Die produkthaltigen Fraktionen werden gesammelt, das Lösemittel wird im Vakuum abgedampft und das erhaltene Produkt wird im Hochvakuum getrocknet. Man erhält 2,34 g (86% d.Th.) der Titelverbindung als hellbeige Kristalle.

Schmp.: ab 207°C (Zers.)
$R_f$ = 0,40 (Acetonitril : Wasser 9:1)
MS (DCI, $NH_3$): m/z = 302 $(M+H)^+$, 319 $(M+NH_4)^+$
$^1$H-NMR (200 MHz, $D_6$-DMSO): $\delta$ = 1,85 (s, 3H, $CH_3CO$); 3,42 (t, J = 6,5 Hz, 2H, $CH_2N$); 3,78 (dd, J = 7, 9 Hz, 1H, H-4 trans); 4,15 (dd, J = 9, 10 Hz, 1H, H-4 cis); 4,75 (m, 1H, H-5); 6,52 (d, J = 10 Hz, 1H, H-3'); 7,32 (d, J = 10 Hz, 1H, H-4'); 7,73 (d, J = 2 Hz, 1H, H-5'); 7,80 (dd, J = 2, 10 Hz, 1H, H-7'); 7,91 (d, J = 10 Hz, 1H, H-8'); 8,27 (m, 1H, CONH); 11,73 (bs, 1H, NH).

Methode B:

Eine gerührte Suspension von 652 mg (1,90 mmol) der Verbindung aus Beispiel 2 in 20 ml wasserfreiem Methanol wird mit 66 mg (0.20 mmol) Caesiumcarbonat versetzt und 1 h bei Raumtemperatur gerührt. Das Lösemittel wird im Vakuum abgedampft und der Rückstand wird mit 30 ml Ether verrührt. Der Niederschlag wird durch Filtration abgetrennt, mit 25 ml Wasser und 5 ml Ether gewaschen und im Hochvakuum getrocknet. Man erhält 358 mg (57%) der Titelverbindung als helle Kristalle.

Schmp.: 232-233°C

Die übrigen physikalischen Daten sind identisch mit der nach Methode A erhaltenen Verbindung.

**Beispiel 2**

(5S)-5-[(Bisacetyl)aminomethyl]-3-(2-oxo-1,2-dihydro-chinolin-6-yl)-oxazolidin-2-on

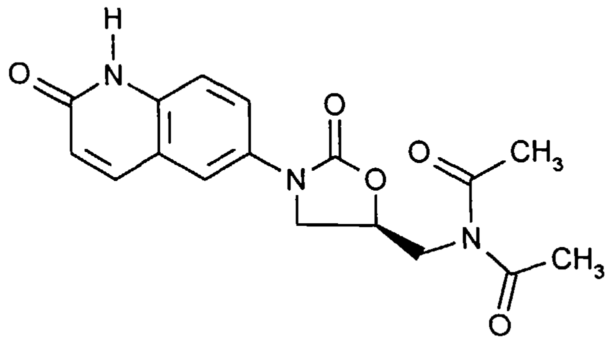

Eine gerührte Suspension von 5,45 g (10,53 mmol) des wasserfreien N-Oxids aus Beispiel VII in 50 ml Essigsäureanhydrid wird 24 h zum Rückfluß erhitzt, wobei eine klare Lösung entsteht. Das Gemisch darf abkühlen und wird am Rotationsverdampfer zur Trockene eingeengt. Der Rückstand wird mit 30 ml Toluol versetzt und erneut zur Trockene eingeengt. Dieser Vorgang wird noch zweimal wiederholt, dann wird das Rohprodukt durch Filtration an 10 g Kieselgel (Dichlormethan : Methanol 95:5) und durch Chromatographie an 100 g Kieselgel (Acetonitril : Wasser 98:2) gereinigt. Man erhält 1,84 g (58%) der Titelverbindung.

Schmp.: 135-137°C
$R_f$ = 0,33 (Dichlormethan : Methanol 9.1) / 0,48 (Acetonitril : Wasser 95:5)
MS (DCI, $NH_3$) m/z = 344 $(M+H)^+$
$^1$H-NMR (200 MHz, $D_6$-DMSO) $\delta$ = 2,38 (s, 6H, $CH_3CO$); 3,84 (dd, J = 7, 9 Hz, 1H- H-4 trans); 4,10 (m, 2H, $CH_2N$); 4,20 (dd, J = 9, 10 Hz, 1H, H-4 cis); 4,82 (m, 1H, H-5); 6,52 (d, J = 10 Hz, 1H, H-3'); 7,33 (d, J = 10 Hz, 1H, H-4'); 7,78 (m, 2H; H-5', H-7'); 7,92 (d, J = 10 Hz, 1H, H-8'); 11,78 (bs, 1H, NH). IR (KBr) $\nu$ = 3449, 1735, 1701, 1654, 1560, 1507, 1437 $cm^{-1}$

**Beispiel 3**

(5S)-3-(1-Methyl-2-oxo-1,2-dihydro-chinolin-6-yl)-5-acetylaminomethyloxazolidin-2-on

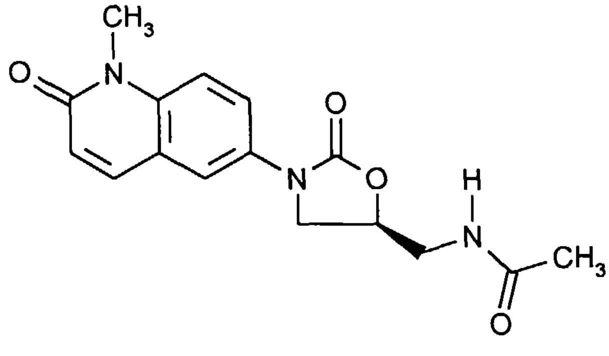

Eine Suspension von 100 mg (0,33 mmol) der Verbindung aus Beispiel 1 und 1,36 mg (0,99 mmol) Kaliumcarbonat in 4 ml wasserfreiem DMF wird mit 67 µl (1,73 mmol) Jodmethan versetzt und 1 h bei 70°C gerührt. Dann wird das Reaktionsgeinisch zur Trockne eingeengt, der Rückstand wird mit einem Gemisch aus 20 ml Wasser und 10 ml Dichlormethan versetzt, die organische Phase wird abgetrennt und die wäßrige Phase wird mehrmals mit Dichlormethan extrahiert. Die vereinigten Extrakte werden über $MgSO_4$ getrocknet. Das Lösemittel wird im Vakuum abgedampft, und der Rückstand wird durch Chromatographie an 10 g Kieselgel (Dichlormethan : Methanol 95:5) gereinigt. Man erhält 60

mg (59%) der Titelverbindung als farblose Kristalle.

Schmp.: 252°C
$R_f$ = 0,29 (Dichlormethan : Methanol 9:1)
MS (DCI, $NH_3$) m/z = 316 $(M+H)^+$
$^1$H-NMR (200 MHz, $D_6$-DMSO) δ = 1,85 (s, 3H, $CH_3CO$); 3,44 (m, 2H, $CH_2N$); 3,60 (s, 3H, $NCH_3$); 3,81 (dd, J = 7, 9 Hz, 1H, H-4 trans); 4,17 (dd, J = 9, 10 Hz, 1H, H-4 cis); 4,78 (m, 1H, H-5); 6,65 (d, J = 10 Hz, 1H, H-3'); 7,59 (d, J = 10 Hz, 1H, H-4'); 7,80 (d, J = 1,5 Hz, 1H, H-5'); 7,92 (m, 2H, H-7', H-8'); 8,28 (t, J = 6 Hz, 1H, CONH).
IR (KBr): ν = 3291, 1740, 1664, 1560, 1449, 1231, 1116, 812, 536 $cm^{-1}$

Wie für Beispiel 3 beschrieben erhielt man durch Alkylierung der Verbindung aus Beispiel 1 mit den entsprechenden Alkyliodiden die in Tabelle 1 aufgeführten Produkte:

**Tabelle 1:**

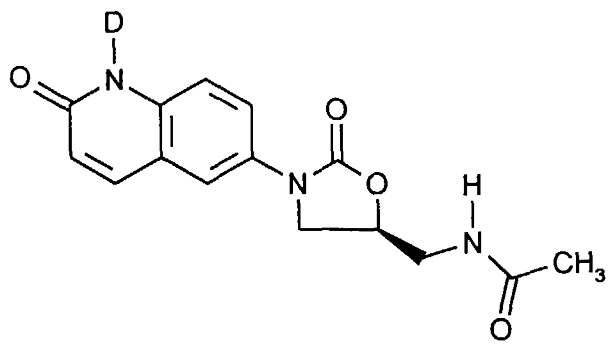

| Bsp.-Nr. | D | Ausbeute (% d.Th.) | Schmp. (°C) | $R_f$ ($CH_2Cl_2$:MeOH) (Verhältnis) | MS (DCI, $NH_3$), m/z $(M+H)^+$ |
|---|---|---|---|---|---|
| 4 | $CH_2CH_3$ | 45 | 196 | 0,39, (9:1) | 330 |
| 5 | $CH(CH_3)_2$ | 21 | 189 | 0,25, (95:5) | 344 |
| 6 | $CH_2CN$ [a)] | 32 | 185 | 0,13, (9:1) | 341 |
| 7 | $CH_2CH_2OH$ | 51 | 174 | 0,57, (85:15) | 346 |
| 8 | $CH_2Ph$ [b)] | 26 | 129 | 0,28, (9:1) | 392 |

a) mit $ClCH_2CN$ b) mit $BrCH_2Ph$

**Beispiel 9**

(5S)-3-(1-[Acetamido-2-yl]-2-oxo-1,2-dihydro-chinolin-6-yl)-5-acetylaminomethyl-oxazolidin-2-on

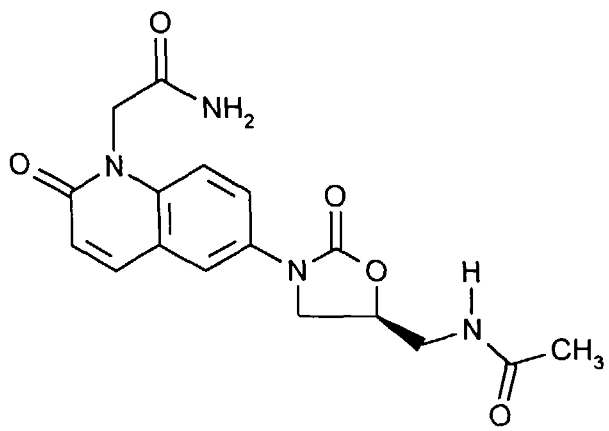

Eine Lösung von 10 mg (0,03 mmol) der Cyanoverbindung aus Beispiel 5 in 0,2 ml Aceton wird mit 60 µl (0.06 mmol) einer 1M wässrigen Kaliumcarbonat-Lösung und mit 40 µl (0,18 mmol) 30% $H_2O_2$ versetzt und 2 h bei Raumtemperatur gerührt. Danach wird die Reaktionsmischung mit 1 ml Toluol versetzt, im Vakuum zur Trockne eingedampft, und der Rückstand wird durch Chromatographie an 1 g Kieselgel (Dichlormethan : Methanol 95:5) gereinigt. Man erhält 7,2 mg (66%) der Titelverbindung als farblose Kristalle.

$R_f$ = 0,11 (Dichlormethan : Methanol 9:1)
MS (DCI, $NH_3$) m/z = 359 $(M+H)^+$, 376 $(M+NH_4)^+$
$^1$H-NMR (200 MHz, $D_6$-DMSO): $\delta$ = 1,85 (s, 3H, $COCH_3$); 3,45 (m, 2H, $CH_2N$); 3,79 (dd, J = 6, 9 Hz, 1H, H-4 trans); 4,18 (dd, J = 9, 10 Hz, 1H, H-4 cis); 4,77 (m, 1H, H-5); 4,88 (s, 2H, $NCH_2CON$); 6,65 (d, J = 10 Hz, 1H, H-3'); 7,27 (bs, 1H, $CONH_2$); 7,32 (d, J = 10 Hz, 1H, H-4'); 7,70 (bs, 1H, $CONH_2$); 7,85 (m, 2H, H-5', H-7'); 7,96 (d, J = 10 Hz, 1H, H-8'); 8,28 (t, J= 6,5 Hz, 1H, CONHCH$_2$).

**Beispiel 10**

(5S)-3-(1-Hydroxymethyl-2-oxo-1,2-dihydro-chinolin-6-yl)-5-acetaminomethyl-oxazolidin-2-on

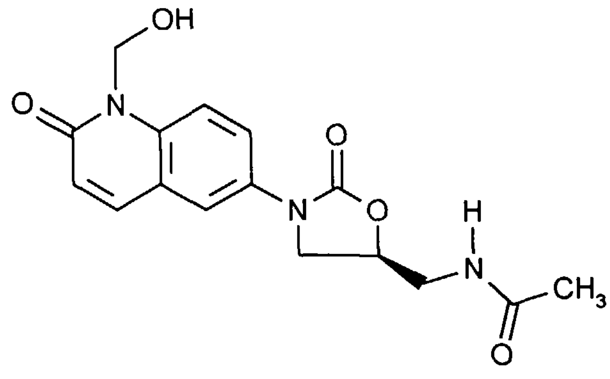

Eine gerührte Suspension von 100 mg (0,33 mmol) der Verbindung aus Beispiel 1 in 3,3 ml (3,3 mmol) 30%iger wäßriger Formalinlösung wird 2 h zum Rückfluß erhitzt. Danach wird im Vakuum zur Trockne eingedampft, der Rückstand mit 10 ml Toluol versetzt und erneut eingedampft. Der zurückbleibende Feststoff wird mit 5 ml Ether verrieben, abgesaugt und im Hochvakuum getrocknet. Man erhält 104 mg (95%) der Titelverbindung als helles Pulver.

**Beispiel 11**

(5S)-3-(1-N,N-[Dimethylamino-methyl]-2-oxo-1,2-dihydro-chinolin-6-yl)-5-acetaminomethyl-oxazolidin-2-on

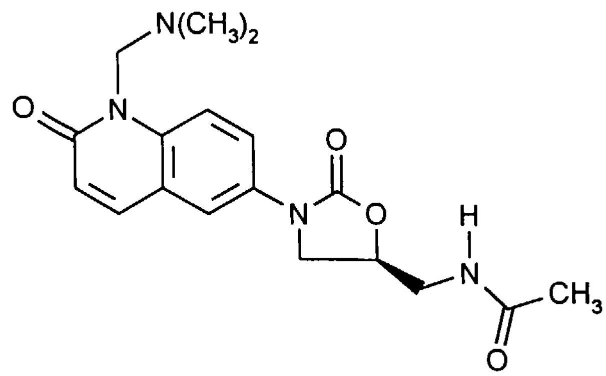

500 mg (0,48 mmol) der Verbindung aus Beispiel 1 werden in 5 ml heißem Ethanol gelöst, mit 0,36 ml 30%iger wäßriger Formalinlösung und 46 µl (0,53 mmol) 51%iger wäßriger Dimethylamin-Lösung versetzt und 8 h zum Rückfluß erhitzt. Die Aufarbeitung erfolgte wie für Beispiel 9 beschrieben. Man erhält 139 mg (81%) der Titelverbindung als Feststoff.

**Beispiel 12**

(5S)-3-(1-Methansulfonyl-2-oxo-1,2-dihydro-chinolin-6-yl)-5-acetylaminomethyl-oxazolidin-2-on

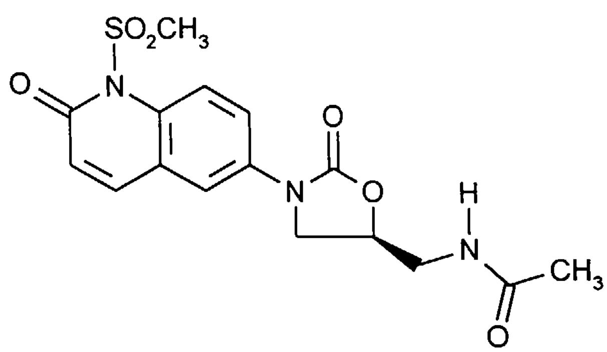

Eine auf 0°C gekühlte, gerührte Lösung von 100 mg (0,33 mmol) der Verbindung aus Beispiel 1 und 0,56 ml (3,96 mmol) Triethylamin in 10 ml wasserfreiem Dichlormethan wird langsam mit 0,27 ml (3,44 mmol) Methansulfonsäurechlorid versetzt. Man rührt 4 h bei 20°C nach und rührt das Gemisch in 5 ml Eiswasser ein. Die organische Phase wird abgetrennt, mit 5 ml gesättigter $NaHCO_3$-Lösung gewaschen und über $MgSO_4$ getrocknet. Das Lösemittel wird im Vakuum eingedampft und der Rückstand wird durch Chromatographie an 5 g Kieselgel (Dichlormethan : Methanol 95:5) gereinigt. Man erhält 224 mg (20%) der Titelverbindung als farblose Kristalle.

Schmp.: 171-173°C
$R_f$ = 0,72 (Acetonitril : Wasser 95:5)
MS (FAB) m/z = 380 $(M+H)^+$ 402 $(M+Na)^+$
$^1$H-NMR (200 MHz, $D_6$-DMSO) $\delta$ = 1,84 (s, 3H, $COCH_3$); 3,48 (t, J = 6 Hz, 2H, $CH_2N$); 3,79 (s, 3H, $CH_3SO_2$); 3,90 (dd, J = 7, 9 Hz, 1H, H-4 trans); 4,27 (dd, J = 9, 9 Hz, 1H, H-4 cis); 4,80 (m, 1H, H-5); 7,42 (d, J = 10 Hz, 1H, H-3'); 8,03 (m, 2H, H-4', H-5'); 8,30 (m, 7H, H-7', CONH); 8,59 (d, J = 10 Hz, 1H, H-8').
IR (KBr) $\nu$ = 3338, 1734, 1654, 1600, 1549, 1517, 1364, 1170, 1140, 986, 813, 528 $cm^{-1}$

**Beispiel 13**

(5S)-3-(1-[3-Chlorbenzoyl]-2-oxo-1,2-dihydro-chinolin-6-yl)-acetaminomethyl-oxazolidin-2-on

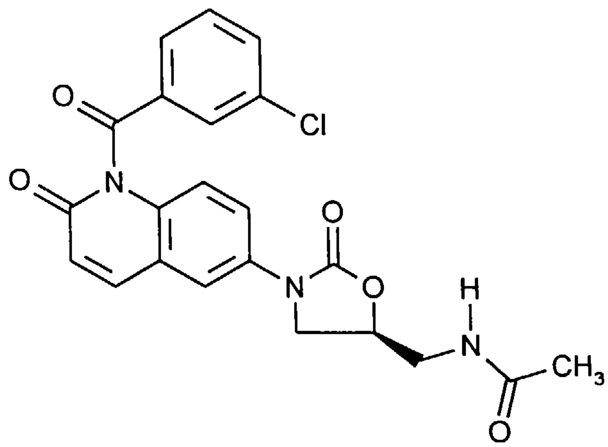

Zu einer gerührten, auf 0°C gekühlten Lösung von 150 mg (0,50 mmol) der Verbindung aus Beispiel 1 und 0,21 ml (1,50 mmol) Triethylamin in 5 ml wasserfreiem DMF gibt man langsam 175 mg (1,00 mmol) 3-Chlorbenzoylchlorid. Man rührt 2 h bei 0°C nach, verdünnt mit 20 ml Wasser und 50 ml Dichlormethan, trennt die organische Phase ab, extrahiert die wäßrige Phase mehrfach mit 5 ml Dichlormethan und trocknet die vereinigten organischen Extrakte über $MgSO_4$. Nach Abdampfen des Lösemittels im Vakuum, Reinigen des Rohproduktes durch Chromatographie an 15 g Kieselgel (Dichlormethan : Methanol 95:5) und Verreiben des Rückstands mit Ether erhält man 95 mg (43%) der Titelverbindung als Kristalle.

Schmp.: 172-174°C
$R_f$ = 0,47 (Dichlormethan : Methanol 9:1)
MS (DCI, $NH_3$): m/z = 440 $(M+H)^+$
$^1$H-NMR (300 MHz, $D_6$-DMSO): $\delta$ = 1,86 (s, 3H, $COCH_3$); 3,50 (t, J = 6 Hz, 2H, $CH_2N$); 3,92 (dd, J = 7, 9 Hz, 1H, H-4 trans); 4,30 (dd, J = 9, 10 Hz, 1H, H-4 cis); 4,82 (m, 1H, H-5); 7,56 (d, J = 10 Hz, 1H, H-3'); 7,65 - 8,35 (m, 8H, H-arom., CONH); 8,58 (d, J = 9 Hz, 1H, H-8').
IR (KBr): $\nu$ = 3284, 1740, 1654, 1560, 1517, 1424, 1258, 1216, 888, 740 $cm^{-1}$

**Beispiel 14**

(5S)-5-(N-Acetyl,N-(2-cyanoethyl)aminomethyl-3-(2-oxo-1,2-dihydro-chinolin-6-yl)-oxazolidin-2-on

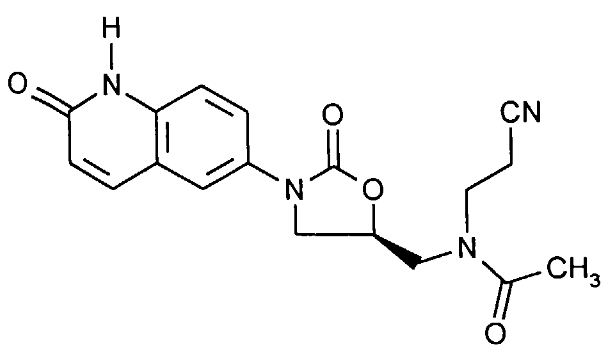

Zu einer gerührten, auf 0°C gekühlten Suspension von 16 mg (0.40 mmol) Natriumhydrid (60% in Öl) in 2 ml wasserfreiem DMF gibt man portionsweise 100 mg (0,33 mmol) der Verbindung aus Beispiel 1 und rührt bis zum Ende der

Wasserstoffentwicklung. Zu der entstandenen klaren Lösung tropft man innerhalb von 5 min 65 µl (0,99 mmol) Acrylnitril. Das Kühlbad wird entfernt, und nach 15 min. wird die Reaktion durch Zugabe von 1 ml 1 N Zitronensäure abgebrochen. Das Reaktionsgemisch wird in eine Mischung aus 10 ml Dichlormethan und 5 ml Wasser gegossen und gut durchgerührt. Die organische Phase wird abgetrennt, die Wasserphase wird mehrmals mit 5 ml Dichlormethan extrahiert und die vereinigten Extrakte werden über $MgSO_4$ getrocknet. Das Lösemittel wird im Vakuum abgedampft und der Rückstand durch Chromatographie an 5 g Kieselgel (Dichlormethan : Methanol 95:5) gereinigt. Man erhalt 30 mg (26%) der Titelverbindung als farblose Kristalle.

Schmp.: 124-125°C
$R_f$ = 0,17 (Dichlormethan : Methanol 9:1)
MS (FAB) m/z = 355 $(M+H)^+$
$^1$H-NMR (250 MHz, $D_6$-DMSO) $\delta$ =2,08, 2,12 (s, 3H, $COCH_3$); 2,70- 2,90 (m, 2H, $CH_2CN$); 3,5 - 3,9 (m, 5H, $CH_2N$, H-4 trans); 4,15 (dd, J = 9, 10 Hz, 1H, H-4 cis); 4,90 (m, 1H, H-5); 6,52 (d, J = 10 Hz, 1H, H-3'); 7,31 (d, J = 10 Hz, 1H, H-4'); 7,80 (m, 2H, H-5', H-7'); 7,91 (dd, J = 2, 10 Hz, 1H, H-8'); 11,90 (bs, 1H, NH).

In Analogie zu Beispiel 14 erhält man das in Tabelle 2 beschriebene Michaeladdukt:

**Tabelle 2:**

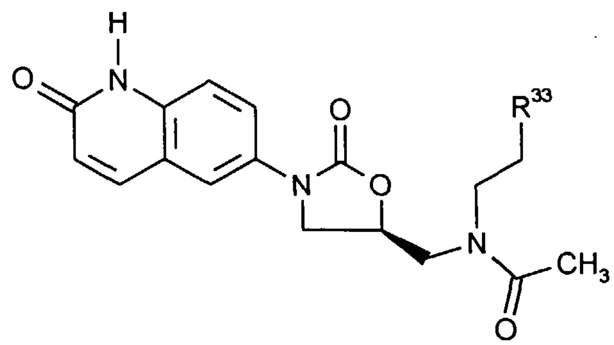

| Bsp.-Nr. | $R^{33}$ | Ausbeute (% d.Th.) | $R_f$ / Laufmittel (Verhältnis) | MS (FAB), m/z $(M+H)^+$ |
|---|---|---|---|---|
| 15 | $COOCH_3$ | 18 | 0,14, I (9:1) | 388 |

**Beispiel 16**

(5S)-3-(1-[N,N'-Dimethyl-ureido-carbonyl]-2-oxo-1,2-dihydro-chinolin-6-yl)-5-acetylaminomethyl-oxazolidin-2-on

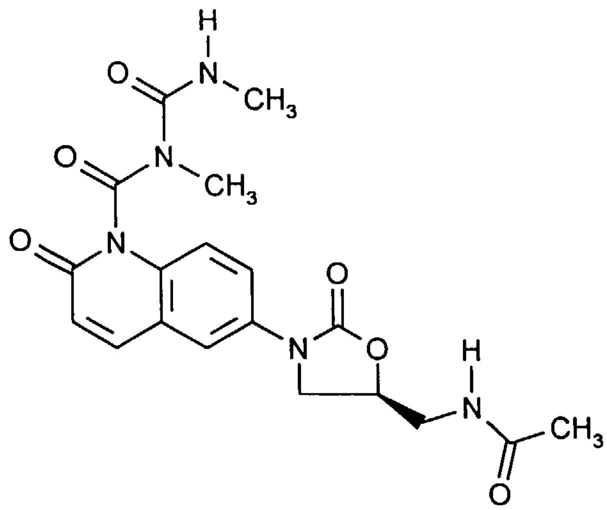

Eine Suspension von 100 mg (0,33 mmol) der Verbindung aus Beispiel 1 in 1,8 ml DMF wird mit 0,58 ml (10,0 mmol) Methylisocyanat und 0,69 ml (5,0 mmol) Triethylamin versetzt und 100 h bei 50°C gerührt. Danach darf die Reaktionsmischung abkühlen und die flüchtigen Bestandteile werden im Vakuum entfernt. Der Rückstand wird durch Chromatographie an 25 g Kieselgel (Dichlormethan : Methanol 95:5) gereinigt. Die produkthaltigen Fraktionen werden gesammelt und das Lösemittel wird im Vakuum abgedampft. Der Rückstand wird in 0,2 ml Dichlormethan : Methanol 95:5 gelöst und durch langsame Zugabe von 2 ml Ether und 2 ml Pentan gefallt. Man erhält 22 mg (19%) der Titelverbindung als helle Kristalle.

Schmp.: ab 112°C (Zers.)
$R_f$ = 0,14 (Dichlormethan : Methanol 9:1)
MS (FAB) m/z == 416 $(M+H)^+$, 438 $(M+Na)^+$
$^1$H-NMR (200 MHz, $D_6$-DMSO): $\delta$ = 1,85 (s, 3H, $COCH_3$); 2,85 (m, 3H, $CH_3N$); 2,96 (s, 3H, $CH_3N$); 3,44 (t, J = 5 Hz, 2H, $CH_2N$); 3,81 (dd, J = 6, 9 Hz, 1H, H-4 trans); 4,19 (dd, J = 9, 10 Hz, 1H, H-4 cis); 4,78 (m, 1H, H-5); 5,73 (bs, 1H, CO<u>NH</u>$CH_3$); 6,72 (d, J = 10 Hz, 1H, H-3'); 7,30 (d, J = 10 Hz, H-4'); 7,90 (m, 2H, H-5', H-7'); 8,12 (d, J = 10 Hz, 1H, H-8'); 8,28 (t, J = 6 Hz, 1H, CO<u>NH</u>$CH_2$).

**Beispiel 17**

(5S)-3-(1-Methyl-2-oxo-1,2-dihydro-chinolin-6-yl)-5-thioacetylaminomethyloxazolidin-2-on

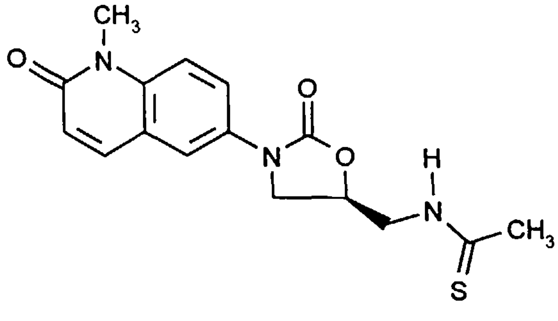

Eine gerührte Lösung von 125 mg (0,40 mmol) der Verbindung aus Beispiel 3 und 162 mg (0,40 mmol) Lawessons

Reagenz in 5 ml wasserfreiem 1,2-Dimethoxyethan wurde 2 h auf 100°C erwärmt. Danach durfte das Reaktionsgemisch abkühlen, man setzte 20 g Kieselgel zu und dampfte das Lösungsmittel im Vakuum ab. Der Rückstand wurde auf eine Säule gegeben und durch Chromatographie an 200 g Kieselgel (Ethylacetat) gereinigt. Man erhielt 22 mg (17 %) der Titelverbindung als helle Kristalle.

Schmp.: 158 - 160°C
$R_f$ = 0,46 (Dichlormethan : Methanol 9 : 1)
MS (DCI, $NH_3$) m/z = 332 $(M+H)^+$
$^1$H-NMR (200 MHz, $D_6$-DMSO): $\delta$ = 2,45 (s, 3H, $CH_3CS$); 3,61 (s, 3H, $NCH_3$); 3,8 - 4,0 (m, 3H, $CH_2N$, H-4 trans); 4,22 (dd, J = 9, 10 Hz, 1H, H-4 cis); 5,00 (m, 1H, H-5); 6,65 (d, J = 10 Hz, 1H, H-3); 7,58 (d, J = 10 Hz, 1H, H-4'); 7,82 (d, J = 1,5 Hz, 1H, H-5'); 7,9 (m, 2H, H-7', H-8'); 10,41 (bt, 1H, CONH).

**Patentansprüche**

1. Oxazolidinon-Verbindungen der allgemeinen Formel (I)

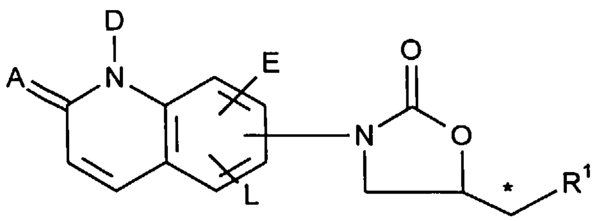

(I) ,

in welcher

| | |
|---|---|
| A | für ein Sauerstoff- oder ein Schwefelatom steht, |
| D | für Wasserstoff oder für Cycloalkyl mit 3 bis 6 Kohlenstoffatomen steht, oder<br>für geradkettiges oder verzweigtes Alkoxycarbonyl mit bis zu 4 Kohlenstoffatomen steht, oder<br>für geradkettiges oder verzweigtes Alkyl oder Alkenyl mit jeweils bis zu 9 Kohlenstoffatomen steht, das gegebenenfalls durch Cyano, Trifluormethyl, Halogen, Hydroxy, Pyridyl, Phenyl, Carboxyl, Carboxamido, geradkettiges oder verzweigtes Alkoxycarbonyl mit bis zu 5 Kohlenstoffatomen, Naphthyl, Cycloalkyl mit 3 bis 6 Kohlenstoffatomen, und/oder durch eine Gruppe der Formel $-(CO)_a-NR^2R^3$,<br>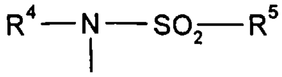 ,<br>$R^6R^7$-N-$SO_2$- oder $R^8$-$S(O)_b$ substituiert ist, worin |
| a | eine Zahl 0 oder 1 bedeutet, |
| $R^2$, $R^3$, $R^4$, $R^6$ und $R^7$ | gleich oder verschieden sind und Wasserstoff, geradkettiges oder verzweigtes Alkyl mit bis zu 5 Kohlenstoffatomen oder Phenyl bedeuten, oder |
| $R^2$ und $R^3$ | gemeinsam mit dem Stickstoffatom einen Morpholinyl-, Thiomorpholinyl-, Pyrrolidinyl-, Piperazinyl- oder Piperidylring bilden, die gegebenenfalls, auch über die freie N-Funktion, durch geradkettiges oder verzweigtes Alkyl oder Acyl mit jeweils bis zu 4 Kohlenstoffatomen substituiert sind, |

| | |
|---|---|
| b | eine Zahl 0, 1 oder 2 bedeutet, |
| $R^5$ und $R^8$ | gleich oder verschieden sind und geradkettiges oder verzweigtes Alkyl mit bis zu 6 Kohlenstoffatomen, Benzyl, Phenyl oder Tolyl bedeuten, oder |
| D | für geradkettiges oder verzweigtes Acyl mit bis zu 5 Kohlenstoffatomen steht, das gegebenenfalls durch Trifluormethyl, Trichlormethyl oder eine Gruppe der Formel $-OR^9$ substituiert ist, worin |
| $R^9$ | Wasserstoff oder geradkettiges oder verzweigtes Alkyl mit bis zu 5 Kohlenstoffatomen bedeutet, das gegebenenfalls durch Phenyl oder Naphthyl substituiert ist, oder |
| D | für eine Gruppe der Formel $-(CT)_d-NR^{10}R^{11}$, $-(CO)_e-NR^{12}-CO-NR^{13}R^{14}$, $-NR^{15}-SO_2R^{16}$, $R^{17}R^{18}-N-SO_2-$, $R^{19}-S(O)_f$ oder $-CO-R^{20}$ steht, worin |
| T | ein Sauerstoff- oder Schwefelatom bedeutet, |
| d und e | gleich oder verschieden sind und die oben angegebene Bedeutung a haben und mit dieser gleich oder verschieden sind, |
| $R^{10}$, $R^{11}$, $R^{12}$, $R^{13}$, $R^{14}$, $R^{15}$, $R^{17}$ und $R^{18}$ | die jeweils oben angegebene Bedeutung von $R^2$, $R^3$ und $R^4$ haben und mit dieser gleich oder verschieden sind, |
| f | die oben angegebene Bedeutung von b hat und mit dieser gleich oder verschieden ist, |
| $R^{16}$ und $R^{19}$ | gleich oder verschieden sind und die oben angegebene Bedeutung von $R^5$ und $R^8$ haben, |
| $R^{20}$ | Aryl mit 6 bis 10 Kohlenstoffatomen bedeutet, das gegebenenfalls durch Halogen substituiert ist, |
| E und L | gleich oder verschieden sind und für Wasserstoff, Carboxy, Halogen, Cyano, Formyl, Trifluormethyl, Nitro, für geradkettiges oder verzweigtes Alkyl mit bis zu 4 Kohlenstoffatomen stehen, |
| $R^1$ | für Azido, Hydroxy oder für eine Gruppe der Formel $-OR^{21}$, $O-SO_2R^{22}$ oder $-NR^{23}R^{24}$ steht, worin |
| $R^{21}$ | geradkettiges oder verzweigtes Acyl mit bis zu 6 Kohlenstoffatomen oder eine Hydroxyschutzgruppe bedeutet, |
| $R^{22}$ | geradkettiges oder verzweigtes Alkyl mit bis zu 5 Kohlenstoffatomen, Phenyl oder Tolyl bedeutet, |
| $R^{23}$ und $R^{24}$ | gleich oder verschieden sind und Cycloalkyl mit 3 bis 6 Kohlenstoffatomen, Wasserstoff, Phenyl oder geradkettiges oder verzweigtes Alkoxy mit bis zu 6 Kohlenstoffatomen, tert.Butoxycarbonyl, Fluorenyloxycarbonyl oder Benzyloxycarbonyl |

bedeuten, oder geradkettiges oder verzweigtes Alkyl mit bis zu 6 Kohlenstoffatomen bedeuten, das gegebenenfalls durch Cyano oder Alkoxycarbonyl mit bis zu 4 Kohlenstoffatomen substituiert ist, oder

$R^{23}$ und/oder $R^{24}$ — eine Gruppe der Formel $-CT'-R^{25}$, $P(O)(OR^{26})(OR^{27})$ oder $-SO_2-R^{28}$ bedeutet, worin

T' — die oben angegebene Bedeutung von T hat und mit dieser gleich oder verschieden ist,

$R^{25}$ — Cycloalkyl mit 3 bis 6 Kohlenstoffatomen, Trifluormethyl oder geradkettiges oder verzweigtes Alkoxy mit bis zu 6 Kohlenstoffatomen, Phenyl, Benzyloxy oder Wasserstoff bedeutet, oder geradkettiges oder verzweigtes Alkyl mit bis zu 6 Kohlenstoffatomen bedeutet, das gegebenenfalls durch geradkettiges oder verzweigtes Alkoxycarbonyl mit bis zu 5 Kohlenstoffatomen, Cyano, Fluor, Chlor, Brom oder Trifluormethyl substituiert ist, oder geradkettiges oder verzweigtes Thioalkyl oder Acyl mit jeweils bis zu 5 Kohlenstoffatomen bedeutet, oder eine Gruppe der Formel $-NR^{29}R^{30}$ bedeutet, worin

$R^{29}$ und $R^{30}$ — gleich oder verschieden sind und Wasserstoff, Phenyl oder geradkettiges oder verzweigtes Alkyl mit bis zu 4 Kohlenstoffatomen bedeuten, oder

$R^{26}$ und $R^{27}$ — gleich oder verschieden sind und Wasserstoff oder geradkettiges oder verzweigtes Alkyl mit bis zu 4 Kohlenstoffatomen bedeuten,

$R^{28}$ — geradkettiges oder verzweigtes Alkyl mit bis zu 4 Kohlenstoffatomen oder Phenyl bedeutet,

und deren Salze und Isomere.

**2.** Verbindungen der allgemeinen Formel (I) nach Anspruch 1 in welcher

A — für ein Sauerstoff- oder ein Schwefelatom steht,

D — für Wasserstoff, Cyclopropyl, Cyclopentyl oder Cyclohexyl steht, oder für geradkettiges oder verzweigtes Alkoxycarbonyl mit bis zu 3 Kohlenstoffatomen steht, oder für geradkettiges oder verzweigtes Alkyl oder Alkenyl mit jeweils bis zu 6 Kohlenstoffatomen steht, das gegebenenfalls durch Cyano, Trifluormethyl, Fluor, Chlor, Brom, Hydroxy, Pyridyl, Phenyl, Carboxyl, Carboxamido, geradkettiges oder verzweigtes Alkoxycarbonyl mit bis zu 4 Kohlenstoffatomen, Naphthyl, Cyclopropyl, Cyclopentyl oder Cyclohexyl und/oder durch eine Gruppe der Formel $-(CO)_a-NR^2R^3$, $R^4-N-SO_2-R^5$, $R^6R^7-N-SO_2-$ oder $R^8-S(O)_b$ substituiert ist, worin

a — eine Zahl 0 oder 1 bedeutet,

$R^2$, $R^3$, $R^4$, $R^6$ und $R^7$ — gleich oder verschieden sind und

Wasserstoff geradkettiges oder verzweigtes Alkyl mit bis zu 3 Kohlenstoffatomen oder Phenyl bedeuten,
oder

| | |
|---|---|
| $R^2$ und $R^3$ | gemeinsam mit dem Stickstoffatom einen Morpholinyl-, Thiomorpholinyl-, Pyrrolidinyl-, Piperazinyl- oder Piperidylring bilden, die gegebenenfalls, auch über die freie N-Funktion, durch geradkettiges oder verzweigtes Alkyl oder Acyl mit jeweils bis zu 3 Kohlenstoffatomen substituiert sind, |
| b | eine Zahl 0, 1 oder 2 bedeutet, |
| $R^5$ und $R^8$ | gleich oder verschieden sind und<br>geradkettiges oder verzweigtes Alkyl mit bis zu 4 Kohlenstoffatomen, Benzyl, Phenyl oder Tolyl bedeuten,<br>oder |
| D | für geradkettiges oder verzweigtes Acyl mit bis zu 4 Kohlenstoffatomen steht, das gegebenenfalls durch Trifluormethyl, Trichlormethyl oder eine Gruppe der Formel $-OR^9$ substituiert ist,<br>worin |
| $R^9$ | Wasserstoff oder geradkettiges oder verzweigtes Alkyl mit bis zu 4 Kohlenstoffatomen bedeutet, das gegebenenfalls durch Phenyl oder Naphthyl substituiert ist,<br>oder |
| D | für eine Gruppe der Formel $-(CT)_d-NR^{10}R^{11}$, $-(CO)_e-NR^{12}-CO-NR^{13}R^{14}$, $-NR^{15}-SO_2R^{16}$, $R^{17}R^{18}-N-SO_2-$, $R^{19}-S(O)_f$ oder $-CO-R^{20}$ steht,<br>worin |
| T | ein Sauerstoff- oder Schwefelatom bedeutet, |
| d und e | gleich oder verschieden sind und<br>die oben angegebene Bedeutung von a haben und mit dieser gleich oder verschieden sind, |
| $R^{10}$, $R^{11}$, $R^{12}$, $R^{13}$, $R^{14}$, $R^{15}$ $R^{17}$ und $R^{18}$ | die jeweils oben angegebene Bedeutung von $R^2$, $R^3$ und $R^4$ haben und mit dieser gleich oder verschieden sind, |
| f | die oben angegebene Bedeutung von b hat und mit dieser gleich oder verschieden ist, |
| $R^{16}$ und $R^{19}$ | gleich oder verschieden sind und die oben angegebene Bedeutung von $R^5$ und $R^8$ haben, |
| $R^{20}$ | Phenyl oder Naphthyl bedeutet, das gegebenenfalls durch Fluor, Chlor oder Brom substituiert ist, |
| E und L | gleich oder verschieden sind und<br>für Wasserstoff, Fluor, Chlor, Brom, Cyano, Methyl oder Trifluormethyl steht, |
| $R^1$ | für Azido, Hydroxy oder für eine Gruppe der Formel $-OR^{21}$, $O-SO_2R^{22}$ oder $-NR^{23}R^{24}$ steht,<br>worin |
| $R^{21}$ | geradkettiges oder verzweigtes Acyl mit bis zu 5 Kohlenstoffatomen |

oder Benzyl bedeutet,

$R^{22}$ geradkettiges oder verzweigtes Alkyl mit bis zu 4 Kohlenstoffatomen, Phenyl oder Tolyl bedeutet,

$R^{23}$ und $R^{24}$ gleich oder verschieden sind und Cyclopropyl, Cyclopentyl, Cyclohexyl, Wasserstoff, Phenyl oder geradkettiges oder verzweigtes Alkoxy mit bis zu 5 Kohlenstoffatomen, tert.Butoxycarbonyl oder Benzyloxycarbonyl bedeuten, oder geradkettiges oder verzweigtes Alkyl mit bis zu 4 Kohlenstoffatomen bedeuten, das gegebenenfalls durch Cyano oder durch geradkettiges oder verzweigtes Alkoxycarbonyl mit bis zu 3 Kohlenstoffatomen substituiert ist,
oder

$R^{23}$ und/oder $R^{24}$ ei ne Gruppe der Formel $-CT'-R^{25}$, $P(O)(OR^{26})(OR^{27})$ oder $-SO_2-R^{28}$ bedeutet,
worin

T' die oben angegebene Bedeutung von T hat und mit dieser gleich oder verschieden ist,

$R^{25}$ Cyclopropyl, Cyclopentyl, Cyclohexyl, Trifluormethyl oder geradkettiges oder verzweigtes Alkoxy mit bis zu 5 Kohlenstoffatomen, Phenyl, Benzyloxy oder Wasserstoff bedeutet, oder geradkettiges oder verzweigtes Alkyl mit bis zu 5 Kohlenstoffatomen bedeutet, das gegebenenfalls durch geradkettiges oder verzweigtes Alkoxycarbonyl mit bis zu 4 Kohlenstoffatomen, Cyano, Fluor, Chlor, Brom oder Trifluormethyl substituiert ist, oder geradkettiges oder verzweigtes Thioalkyl oder Acyl mit jeweils bis zu 4 Kohlenstoffatomen bedeutet, oder
eine Gruppe der Formel $-NR^{29}R^{30}$ bedeutet,
worin

$R^{29}$ und $R^{30}$ gleich oder verschieden sind und Wasserstoff, Phenyl oder geradkettiges oder verzweigtes Alkyl mit bis zu 3 Kohlenstoffatomen bedeuten,

$R^{26}$ und $R^{27}$ gleich oder verschieden sind und Wasserstoff oder geradkettiges oder verzweigtes Alkyl mit bis zu 3 Kohlenstoffatomen bedeuten,

$R^{28}$ geradkettiges oder verzweigtes Alkyl mit bis zu 3 Kohlenstoffatomen oder Phenyl bedeutet,

und deren Salze und Isomere.

**3.** Verbindungen der allgemeinen Formel (I) nach Anspruch 1
in welcher

A für ein Sauerstoff- oder ein Schwefelatom steht,

D für Wasserstoff, Cyclopropyl, Cyclopentyl, Cyclohexyl, Allyl oder für geradkettiges oder verzweigtes Alkyl mit bis zu 5 Kohlenstoffatomen steht, das gegebenenfalls durch Cyano, Hydroxy, Trifluormethyl, Fluor, Chlor, Phenyl, Carboxyl, Carboxamido, geradkettiges oder verzweigtes Alkoxycarbonyl mit bis zu 3 Kohlenstoffatomen, Cyclopropyl, Cyclopentyl oder Cyclohexyl und/oder durch eine Gruppe der Formel $-(CO)_a-NR^2R^3$, $R^4-N-SO_2-R^5$, $R^6R^7N-SO_2-$ oder $R^8-S(O)_b$ substituiert ist,
worin

| | |
|---|---|
| a | eine Zahl 0 oder 1 bedeutet, |
| $R^2$, $R^3$, $R^4$, $R^6$ und $R^7$ | gleich oder verschieden sind und Wasserstoff oder Methyl bedeuten, |
| b | eine Zahl 0, 1 oder 2 bedeutet, |
| $R^5$ und $R^8$ | gleich oder verschieden sind und geradkettiges oder verzweigtes Alkyl mit bis zu 3 Kohlenstoffatomen, Benzyl, Phenyl oder Tolyl bedeuten, oder |
| D | für eine Gruppe der Formel $-(CT)_d-NR^{10}R^{11}$, $-(CO)_e-NR^{12}-CO-NR^{13}R^{14}$, $-NR^{15}-SO_2R^{16}$, $R^{17}R^{18}N-SO_2-$, $R^{19}-S(O)_f$ oder $-CO-R^{20}$ steht, worin |
| T | ein Sauerstoff- oder Schwefelatom bedeutet, |
| d und e | gleich oder verschieden sind und die oben angegebene Bedeutung von a haben und mit dieser gleich oder verschieden sind, |
| $R^{10}$, $R^{11}$, $R^{12}$, $R^{13}$, $R^{14}$, $R^{15}$, $R^{17}$ und $R^{18}$ | die jeweils oben angegebene Bedeutung von $R^2$, $R^3$ und $R^4$ haben und mit dieser gleich oder verschieden sind, |
| f | die oben angegebene Bedeutung von b hat und mit dieser gleich oder verschieden ist, |
| $R^{16}$ und $R^{19}$ | gleich oder verschieden sind und die oben angegebene Bedeutung von $R^5$ und $R^8$ haben, |
| $R^{20}$ | Phenyl oder Naphthyl bedeutet, das gegebenenfalls durch Fluor, Chlor oder Brom substituiert ist, |
| E und L | gleich oder verschieden sind und für Wasserstoff oder Fluor stehen, |
| $R^1$ | für Azido, Hydroxy oder für eine Gruppe der Formel $-OR^{21}$, $O-SO_2R^{22}$ oder $-NR^{23}R^{24}$ steht, worin |
| $R^{21}$ | geradkettiges oder verzweigtes Acyl mit bis zu 4 Kohlenstoffatomen bedeutet, |
| $R^{22}$ | Methyl oder Tolyl bedeutet, |
| $R^{23}$ und $R^{24}$ | gleich oder verschieden sind und Cyclopropyl, Wasserstoff, Phenyl oder geradkettiges oder verzweigtes Alkoxy mit bis zu 4 Kohlenstoffatomen, tert.Butoxycarbonyl oder Benzyloxycarbonyl bedeuten, oder geradkettiges oder verzweigtes Alkyl mit bis zu 3 Kohlenstoffatomen bedeuten, das gegebenenfalls durch Cyano oder Methoxycarbonyl substituiert ist, oder |
| $R^{23}$ und/oder $R^{24}$ | eine Gruppe der Formel $-CT'-R^{25}$ bedeutet, worin |
| T' | die oben angegebene Bedeutung von T hat und mit dieser gleich oder |

verschieden ist,

| | |
|---|---|
| $R^{25}$ | Cyclopropyl, Cyclopentyl, Cyclohexyl, Trifluormethyl oder geradkettiges oder verzweigtes Alkoxy mit bis zu 4 Kohlenstoffatomen, Phenyl, Benzyloxy oder Wasserstoff bedeutet, oder<br>geradkettiges oder verzweigtes Alkyl mit bis zu 4 Kohlenstoffatomen bedeutet, das gegebenenfalls durch geradkettiges oder verzweigtes Alkoxycarbonyl mit bis zu 3 Kohlenstoffatomen, Cyano, Fluor, Chlor, Brom oder Trifluormethyl substituiert ist, oder<br>geradkettiges oder verzweigtes Thioalkyl oder Acyl mit jeweils bis zu 3 Kohlenstoffatomen bedeutet, oder<br>eine Gruppe der Formel $-NR^{29}R^{30}$ bedeutet,<br>worin |
| $R^{29}$ und $R^{30}$ | gleich oder verschieden sind und Wasserstoff, Phenyl, Methyl oder Ethyl bedeuten, |

und deren Salze und Isomere.

4. Verbindungen der allgemeinen Formel (I) nach Anspruch 1 in welcher

E und L für Wasserstoff stehen und der Oxazolidinonrest in den Positionen 6 oder 7 an den 1,2-Dihydrochinolinylring angebunden ist.

5. Verfahren zur Herstellung von Verbindungen der allgemeinen Formel (I) nach Anspruch 1, dadurch gekennzeichnet, daß man

[A] N-Oxide der allgemeinen Formel (II)

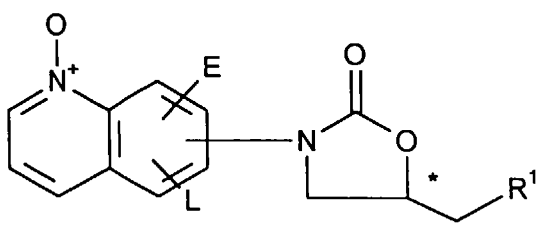

(II) ,

in welcher

| | |
|---|---|
| E und L | die in Anspruch 1 angegebene Bedeutung haben,<br>und |
| $R^1$ | die in Anspruch 1 angegebene Bedeutung hat, vorzugsweise aber für den Rest der Formel $-NH-CO-NR^{31}$ steht,<br>worin |
| $R^{31}$ | geradkettiges oder verzweigtes Alkyl mit bis zu 4 Kohlenstoffatomen bedeutet, |

durch Umsetzung mit $Ac_2O$ oder $p\text{-}TsCl/K_2CO_3$ in inerten Lösemitteln zunächst in die Verbindungen der allgemeinen Formel (Ia)

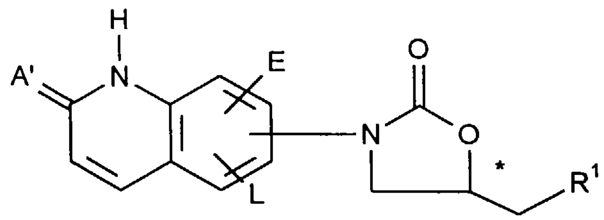

(Ia) ,

in welcher

| | |
|---|---|
| $R^1$, E und L | die oben angegebene Bedeutung haben und |
| A' | für Sauerstoff steht, |

umsetzt,
oder

[B] Verbindungen der allgemeinen Formel (Ib)

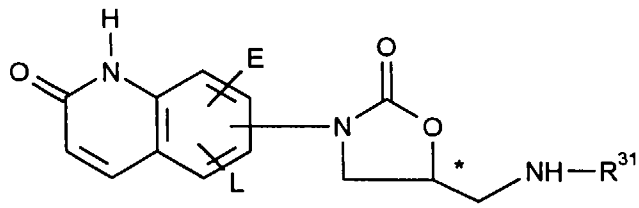

(Ib) ,

in welcher

| | |
|---|---|
| E und L | die oben angegebene Bedeutung haben, |
| $R^{31}$ | für $C_1$-$C_4$-Acyl oder Alkoxycarbonyl steht, |

mit Alkylierungsmitteln der allgemeinen Formel (III)

D'-X (III),

in welcher

| | |
|---|---|
| D' | die in Anspruch 1 angegebene Bedeutung von D hat aber nicht für Wasserstoff steht, und |
| X | für Trifluormethansulfonat oder Halogen steht, |

in inerten Lösemitteln und in Anwesenheit einer Base umsetzt,
und gegebenenfalls die anderen unter $R^1$ aufgeführten Substituenten nach üblichen Methoden einführt,
oder

[C] Verbindungen der allgemeinen Formel (IV)

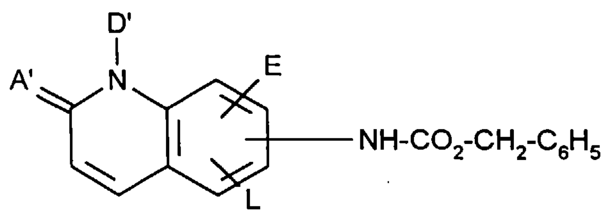

(IV) ,

in welcher

A', D', E und L die oben angegebene Bedeutung haben,

zunächst durch Umsetzung mit Lithiumalkylen in Ethern und R-Glycidylbutyrat der Formel (V)

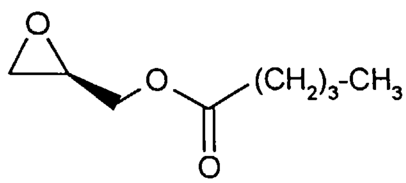

(V)

in die Verbindungen der allgemeinen Formel (Ic)

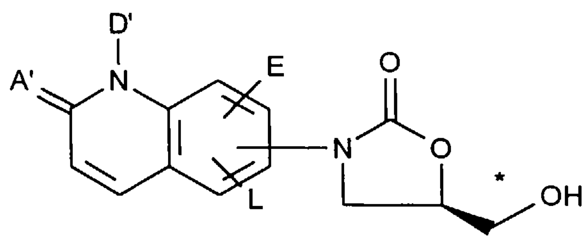

(Ic) ,

in welcher

A', D', E und L die oben angegebene Bedeutung haben,

überführt,
die durch Umsetzung mit $(C_1-C_4)$-Alkyl- oder Phenylsulfonsäurechloriden in inerten Lösemitteln und in Anwesenheit einer Base in die entsprechenden Verbindungen der allgemeinen Formel (Id)

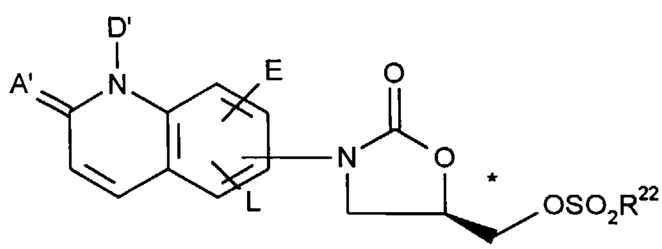

(Id) ,

in welcher

A', D', E und L die oben angegebene Bedeutung haben und

$R^{22}$ die in Anspruch 1 angegebene Bedeutung hat,

überführt,
anschließend mit Natriumazid in inerten Lösemitteln die Azide der allgemeinen Formel (Ie)

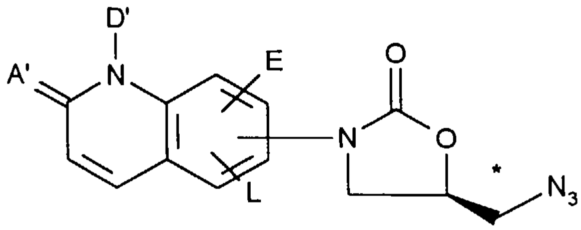

(Ie) ,

in welcher

A', D', E und L die oben angegebene Bedeutung haben,

herstellt,
die in einem weiteren Schritt durch Umsetzung mit Alkylphosphiten oder $PPh_3$ in inerten Lösemitteln und mit Säuren in die Amine der allgemeinen Formel (If)

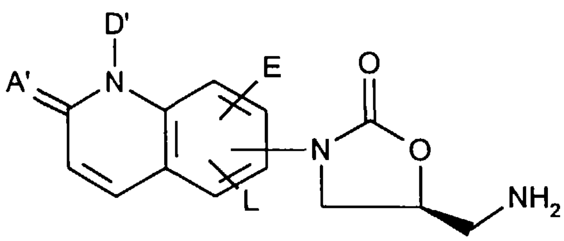

(If) ,

in welcher

A', D', E und L die oben angegebene Bedeutung haben,

überführt,
und durch Umsetzung mit Acetanhydrid oder anderen Acylierungsmitteln der allgemeinen Formel (VI)

$$R^{32}\text{-CT'-}R^{25} \qquad \text{(VI)},$$

in welcher

$R^{25}$ die in Anspruch 1 angegebene Bedeutung hat und
T' die in Anspruch 1 angegebene Bedeutung von T hat und
$R^{32}$ für Halogen oder für den Rest $-OCOR^{25}$ steht,

in inerten Lösemitteln die Verbindungen der allgemeinen Formel (Ig)

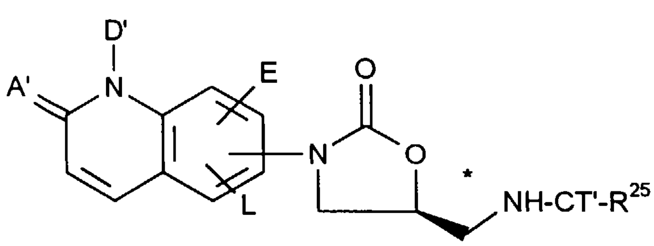

(Ig) ,

in welcher

A', D', E, L, T' und $R^{25}$ die oben angegebene Bedeutung haben

herstellt,
und im Fall A = S beispielsweise Verbindungen der allgemeinen Formel (Ig) einer Schwefelung der Amidfunktion mit Lawessons-Reagenz oder $P_2S_5$ in Toluol oder 1,2-Dimethoxyethan unterzieht.

**6.** Verbindungen der allgemeinen Formel (II)

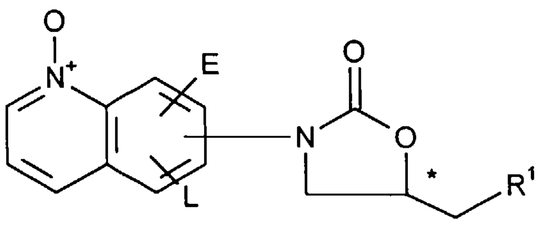

(II) ,

in welcher

$R^1$, E und L die in Anspruch 1 angegebene Bedeutung haben.

**7.** Verbindungen der allgemeinen Formel (IV)

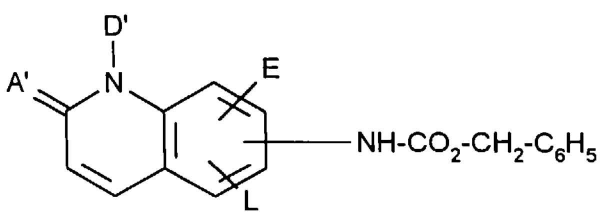

(IV) ,

in welcher

A' für Sauerstoff steht,

D' die in Anspruch 1 angegebene Bedeutung von D hat aber nicht für Wasserstoff steht, und

E und L die in Anspruch 1 angegebene Bedeutung haben.

**8.** Verbindungen der allgemeinen Formel (VII)

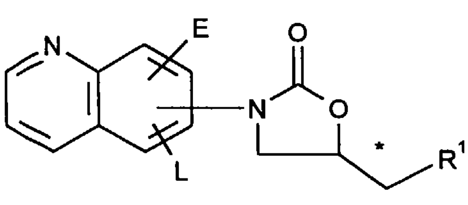

(VII) ,

in welcher

$R^1$, E und L die in Anspruch 1 angegebene Bedeutung haben.

**9.** Verbindungen nach einem der Ansprüche 1 bis 4 zur Verwendung bei der Bekämpfung von Krankheiten.

**10.** Verwendung von Verbindungen nach einem der Ansprüche 1 bis 4 zur Herstellung von Arzneimitteln.

**11.** Arzneimittel enthaltend Verbindungen nach einem der Ansprüche 1 bis 4.